(19) **Europäisches Patentamt European Patent Office Office européen des brevets**

(11) **EP 4 733 379 A1**

(12) **EUROPEAN PATENT APPLICATION**
published in accordance with Art. 153(4) EPC

(43) Date of publication:
**29.04.2026 Bulletin 2026/18**

(21) Application number: **24848813.2**

(22) Date of filing: **04.07.2024**

(51) International Patent Classification (IPC):
*C12M 1/04* (2006.01)    *C12M 1/00* (2006.01)
*C12N 1/00* (2006.01)

(52) Cooperative Patent Classification (CPC):
**C12M 1/00; C12M 1/04; C12N 1/00**

(86) International application number:
**PCT/JP2024/024284**

(87) International publication number:
**WO 2025/028154 (06.02.2025 Gazette 2025/06)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **31.07.2023 JP 2023125057**

(71) Applicant: **FUJIFILM Corporation**
**Tokyo 106-8620 (JP)**

(72) Inventors:
• **SAKAI, Hidekazu**
  **Ashigarakami-gun, Kanagawa 258-8577 (JP)**
• **WAKITA, Taku**
  **Ashigarakami-gun, Kanagawa 258-8577 (JP)**

(74) Representative: **Hoffmann Eitle**
**Patent- und Rechtsanwälte PartmbB**
**Arabellastraße 30**
**81925 München (DE)**

(54) **CULTURE DEVICE AND METHOD OF CONTROLLING CULTURE DEVICE**

(57)    A culture device is a culture device that cultures a microorganism or a cell that uses a raw material gas containing a combustible gas for proliferation or production of an organic substance, the culture device including a culture tank that accommodates a culture solution, a raw material gas supply unit that supplies the raw material gas to the culture tank, and a composition ratio control mechanism that measures a composition ratio of a mixed gas in a space above a liquid surface of the culture solution in the culture tank and supplies an adjustment gas into the space in accordance with the measured composition ratio of the mixed gas to maintain the composition ratio of the mixed gas in the space at a target value outside a preset explosive range, in which, in a case where a circular equivalent diameter of a cross section orthogonal to a height direction of the culture tank is defined as DAm and a maximum height of the liquid surface is defined as H, a dimension of the culture tank satisfies a condition of $H/DAm \geq 2$.

FIG. 17

DIMENSION CONDITION OF CULTURE TANK:
$H/DAm \geq 2$
PREFERABLY
$H/DAm \geq 13$

DIMENSION CONDITION OF TEMPERATURE ADJUSTER:
$Ht \geq H \times 0.3$

**Description**

**BACKGROUND OF THE INVENTION**

1. Field of the Invention

The technology of the present disclosure relates to a culture device and a method for controlling the culture device.

2. Description of the Related Art

In order to suppress greenhouse gas emissions, research on extrication from dependence on petroleum as a raw material is actively conducted worldwide. As part of this research, a technology of using a carbon source other than petroleum as a raw material has been developed in the field of manufacturing products such as chemical products and bioproducts. For example, microorganisms are known to use a carbon source other than petroleum as a raw material for proliferation or production of an organic substance. An example of such a microorganism is hydrogen-oxidizing bacteria. The hydrogen-oxidizing bacteria proliferate by taking in a gas containing carbon dioxide, hydrogen, and oxygen as a raw material, and further efficiently produce organic substances such as alcohol and amino acids by modifying genes of the hydrogen-oxidizing bacteria. The hydrogen-oxidizing bacteria are a general term for chemosynthetic bacteria that convert carbon contained in carbon dioxide into an organic substance by using energy generated by the oxidation of hydrogen, and take the organic substance into the bacteria. The hydrogen-oxidizing bacteria are attracting attention also from the viewpoint that carbon dioxide is a raw material for proliferation or production of an organic substance, and thus carbon dioxide emitted as a greenhouse gas can be effectively used.

JP2564008B and US2019/0316072A disclose a culture device that cultures hydrogen-oxidizing bacteria. The culture device includes a culture tank that accommodates a culture solution. A raw material gas containing carbon dioxide, hydrogen, and oxygen is supplied into the culture tank, and a part of the carbon dioxide, the hydrogen, and the oxygen contained in the raw material gas is dissolved in the culture solution and is consumed for the proliferation of the hydrogen-oxidizing bacteria or the production of the organic substance. The remaining raw material gas is released into a space present above a liquid surface of the culture solution in the culture tank and is exhausted to the outside of the culture tank. Since a mixed gas containing hydrogen, which is a combustible gas, and oxygen may be explosive depending on a composition ratio, JP2564008B and US2019/0316072A disclose a technology of suppressing an explosion.

In JP2564008B, after the supply of oxygen contained in the raw material gas is made independently controllable, a dissolved oxygen concentration in the culture solution is measured, and a supply amount of oxygen to the culture tank is restricted based on the measured dissolved oxygen concentration. In US2019/0316072A, safety is ensured by controlling a component of the raw material gas.

In addition, WO2011/070791A discloses a culture device that cultures a microorganism or the like. WO2011/070791A discloses a technology of improving an oxygen use efficiency by reducing a diameter of bubbles of a raw material gas. JP2013-005754A and WO2006/101074A disclose a technology of improving the solubility of a raw material gas by using a vertically long culture tank as the culture tank. WO2016/117023A discloses a technology of suppressing damage to a microorganism, which is a biomass, in a culture tank, although the raw material gas is not a combustible gas.

**SUMMARY OF THE INVENTION**

In a case of a raw material gas containing a combustible gas, since there is a risk of an explosion of a mixed gas released from the culture solution in the culture tank, a technology of suppressing an explosion is very important. In the method disclosed in JP2564008B and the like, there is room for improvement in safety measures for suppressing an explosion.

The technology of the present disclosure provides a culture device and a method for controlling the culture device, which can improve culture efficiency and safety as compared with the related art in a case of culturing a microorganism or a cell that uses a raw material gas containing a combustible gas for proliferation or for production of an organic substance.

In order to achieve the above object, a first culture device according to the disclosed technology is a culture device that cultures a microorganism or a cell that uses a raw material gas containing a combustible gas for proliferation or production of an organic substance, the culture device comprising: a culture tank that accommodates a culture solution for culturing the microorganism or the cell; a raw material gas supply unit that supplies the raw material gas to the culture tank; a composition ratio control mechanism that measures a composition ratio of a mixed gas in a space above a liquid surface of the culture solution in the culture tank and supplies an adjustment gas into the space in accordance with the measured composition ratio of the mixed gas to maintain the composition ratio of the mixed gas in the space at a target value outside a preset explosive range; a gas circulation path for reusing the mixed gas in the space as the raw material gas; and a dissolution rate control mechanism that measures, during the culture, a combustible gas dissolution rate, which is a proportion of the combustible gas dissolved in the culture solution, to the combustible gas contained in the raw material gas supplied to the culture tank through the gas circulation path or the raw material gas supply unit, and maintains the measured combustible gas dissolution rate at or above a preset target value.

A method for controlling a first culture device according to the disclosed technology is a method for controlling a culture device that cultures a microorganism or a cell and that includes a culture tank that accommodates a culture solution for culturing a microorganism or a cell that uses a raw material gas containing a combustible gas for proliferation or production of an organic substance, and a raw material gas supply unit that supplies the raw material gas containing a combustible gas to the culture tank, the method including: measuring a composition ratio of a mixed gas in a space above a liquid surface of the culture solution in the culture tank; supplying an adjustment gas into the space in accordance with the measured composition ratio of the mixed gas to maintain the composition ratio of the mixed gas in the space at a target value outside a preset explosive range; and measuring, during the culture, a combustible gas dissolution rate, which is a proportion of the combustible gas dissolved in the culture solution, to the combustible gas contained in the raw material gas supplied to the culture tank through a gas circulation path for reusing the mixed gas in the space as the raw material gas or the raw material gas supply unit, and maintaining the measured combustible gas dissolution rate at a preset target value.

A second culture device according to the disclosed technology is a culture device that cultures a microorganism or a cell that uses a raw material gas containing a combustible gas for proliferation or production of an organic substance, the culture device comprising: a culture tank that accommodates a culture solution for culturing the microorganism or the cell; a raw material gas supply unit that supplies the raw material gas to the culture tank; and a composition ratio control mechanism that measures a composition ratio of a mixed gas in a space above a liquid surface of the culture solution in the culture tank and supplies an adjustment gas into the space in accordance with the measured composition ratio of the mixed gas to maintain the composition ratio of the mixed gas in the space at a target value outside a preset explosive range, in which, in a case where a circular equivalent diameter of a cross section orthogonal to a height direction of the culture tank is defined as DAm and a maximum height of the liquid surface is defined as H, a dimension of the culture tank satisfies a condition of H/DAm $\geq$ 2.

A method for controlling a second culture device according to the disclosed technology is a method for controlling a culture device that cultures a microorganism or a cell and that includes a culture tank that accommodates a culture solution for culturing a microorganism or a cell that uses a raw material gas containing a combustible gas for proliferation or production of an organic substance, and a raw material gas supply unit that supplies the raw material gas containing a combustible gas to the culture tank, in which, in a case where a circular equivalent diameter of a cross section orthogonal to a height direction of the culture tank is defined as DAm and a maximum height of the liquid surface is defined as H, a dimension of the culture tank satisfies a condition of H/DAm $\geq$ 2, the method including: measuring a composition ratio of a mixed gas in a space above a liquid surface of the culture solution in the culture tank; and supplying an adjustment gas into the space in accordance with the measured composition ratio of the mixed gas to maintain the composition ratio of the mixed gas in the space at a target value outside a preset explosive range.

A third culture device according to the disclosed technology is a culture device that cultures a microorganism or a cell that uses a raw material gas containing a combustible gas for proliferation or production of an organic substance, the culture device comprising: a culture tank that accommodates a culture solution for culturing the microorganism or the cell; a culture solution tank that stores the culture solution to be supplied to the culture tank; a culture target supply path that supplies the microorganism or the cell to the culture tank; a raw material gas tank that stores the raw material gas; a bubble generation device for mixing the raw material gas with the culture solution before the culture solution is supplied to the culture tank; and a culture solution supply path that is disposed between the culture solution tank and the culture tank, and supplies the culture solution mixed with the raw material gas by the bubble generation device to the culture tank, the culture solution supply path being provided separately from the culture target supply path.

A fourth culture device according to the disclosed technology is a culture device that cultures a microorganism or a cell that uses a raw material gas containing a combustible gas for proliferation or production of an organic substance, the culture device comprising: a culture tank that accommodates a culture solution for culturing the microorganism or the cell; a raw material gas supply unit that supplies the raw material gas to the culture tank; a gas circulation path for reusing a mixed gas in a space above a liquid surface of the culture solution in the culture tank as the raw material gas; and a pressure applying device that is disposed on the gas circulation path and applies a circulation pressure to the mixed gas to circulate the mixed gas, in which the pressure applying device does not have a mechanical movable part, and applies the circulation pressure to the mixed gas by a drive fluid input from the outside to the pressure applying device.

According to the technology of the present disclosure, in a case of culturing a microorganism or a cell that uses a raw material gas containing a combustible gas for proliferation or for production of an organic substance, it is possible to improve safety as compared with the related art.

**BRIEF DESCRIPTION OF THE DRAWINGS**

FIG. 1 is a diagram illustrating a function of a culture device.
FIG. 2 is a diagram illustrating an overall configuration of the culture device.
FIG. 3 is a diagram showing a configuration in which a raw material gas is supplied for each component.
FIG. 4 is a diagram showing a configuration for recovering and reusing a culture solution.
FIG. 5 is a diagram showing a configuration for separating a specific component from the culture solution.
FIG. 6 is a diagram showing a disposition of an outlet of the culture solution.
FIG. 7 is a diagram showing an index of a bubble diameter.
FIG. 8 is a diagram showing a surface tension reducer.
FIG. 9 is a diagram showing a configuration for reusing a waste gas.
FIG. 10 is a diagram illustrating a configuration used for various controls.
FIG. 11 is a diagram illustrating a configuration of composition ratio control of a mixed gas.
FIG. 12 is a diagram illustrating a configuration of dissolution rate control and consumption rate control.
FIG. 13 is a flowchart of a composition ratio control process of a mixed gas.
FIG. 14 is a flowchart of hydrogen dissolution rate control process.
FIG. 15 is a flowchart of hydrogen consumption rate control process.
FIG. 16 is a view showing effects of a first embodiment.
FIG. 17 is a diagram illustrating a culture tank of a second embodiment.
FIG. 18 is a diagram illustrating a first modification example of the culture tank of the second embodiment.
FIG. 19 is a diagram illustrating a second modification example of the culture tank of the second embodiment.
FIG. 20 is a diagram illustrating a third modification example of the culture tank of the second embodiment.
FIG. 21 is a diagram illustrating a fourth modification example of the culture tank of the second embodiment.
FIG. 22 is a diagram illustrating a culture device of a third embodiment.
FIG. 23 is a diagram illustrating a culture device of a fourth embodiment.
FIG. 24 is a diagram illustrating a modification example of the culture device of the fourth embodiment.

**DESCRIPTION OF THE PREFERRED EMBODIMENTS**

[Table of Contents]

1. Example of Common Configuration of Each Embodiment
2. First Embodiment
3. Second Embodiment
4. Third Embodiment
5. Fourth Embodiment

1. Example of Common Configuration of Each Embodiment

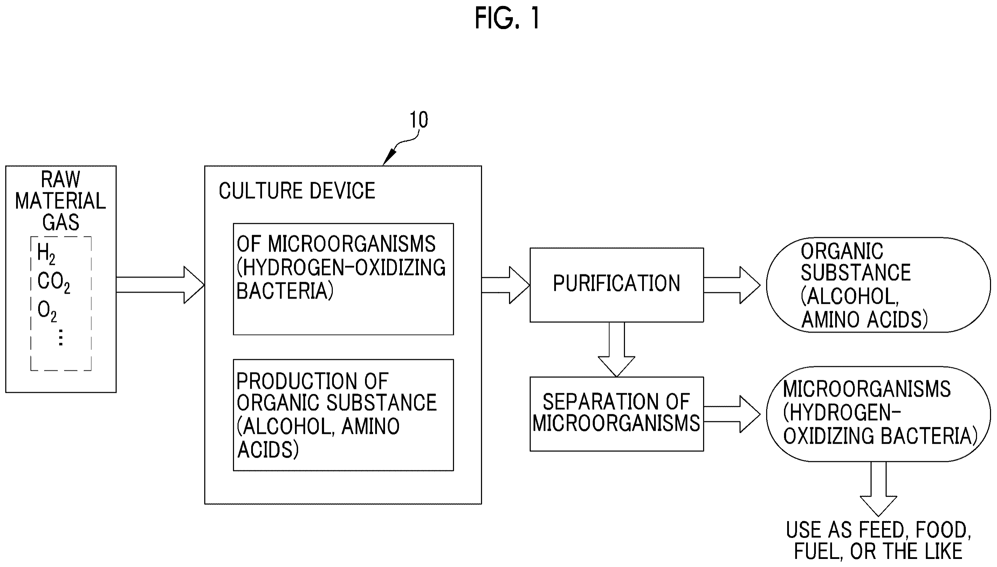

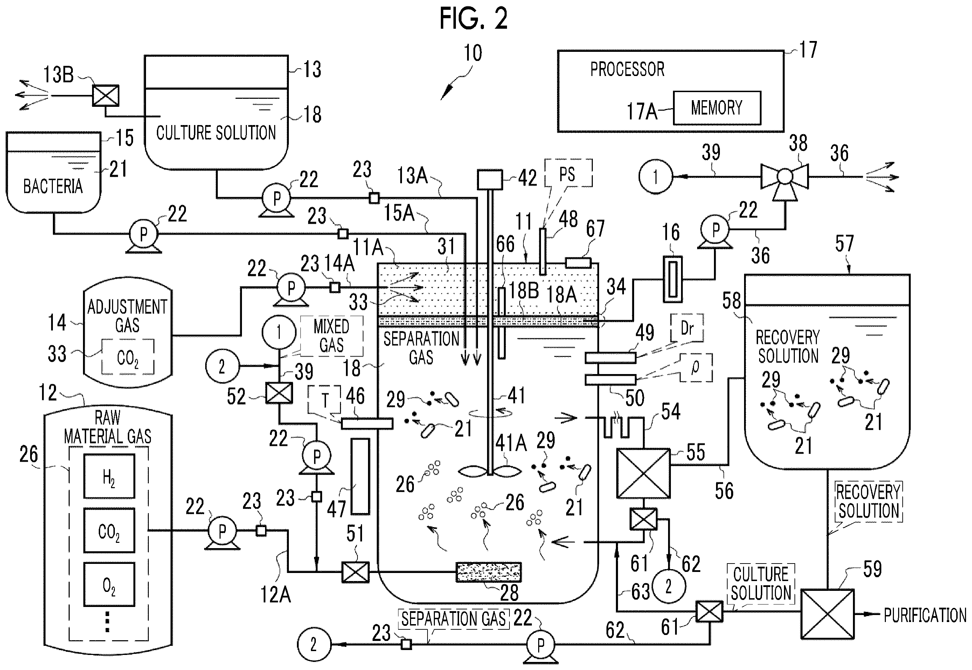

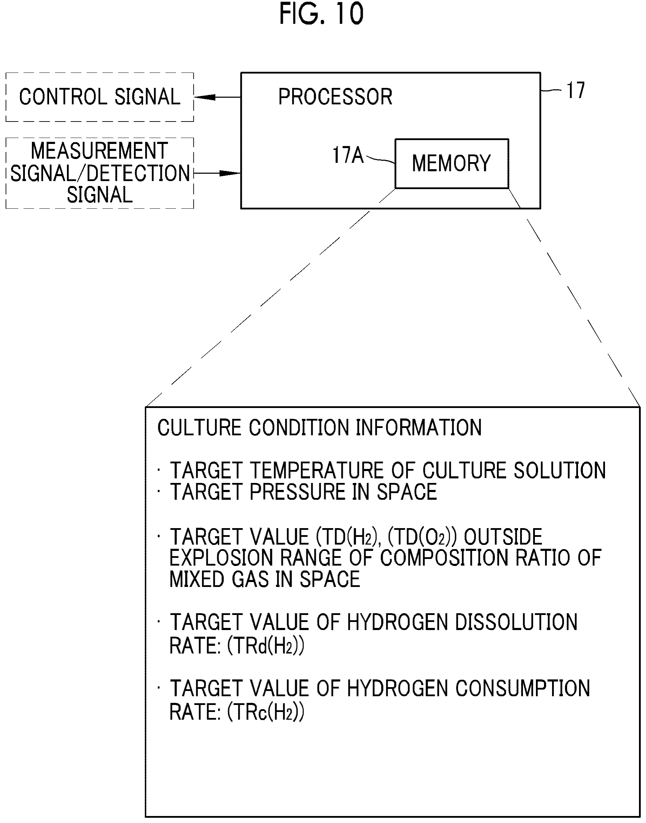

First, an example of a common configuration of the culture device according to each embodiment of the disclosed technology will be described with reference to FIGS. 1 to 9. Then, in FIG. 10 and subsequent figures, each embodiment will be described with the example of the common configuration.
As illustrated in FIG. 1, a culture device 10 according to the disclosed technology cultures a hydrogen-oxidizing bacterium that is a microorganism, as an example of a culture target. A raw material for proliferating the hydrogen-oxidizing bacteria contains an inorganic component such as ammonium sulfate in addition to hydrogen ($H_2$), oxygen ($O_2$), and carbon dioxide ($CO_2$). The hydrogen is an example of a combustible gas. The culture device 10 supplies a raw material gas to a culture solution to proliferate the hydrogen-oxidizing bacteria in the culture solution. In addition, in the culture solution, the hydrogen-oxidizing bacteria efficiently produce organic substances such as alcohol and amino acids by modifying genes of the hydrogen-oxidizing bacteria. The culture solution is purified to extract the organic substance produced in the culture solution. The extracted organic substance is used, for example, in the production of a chemical product, a bioproduct, or the like. In addition, the hydrogen-oxidizing bacteria are separated from the culture solution, and the separated hydrogen-oxidizing bacteria are considered for use as, for example, feed, food, fuel, or the like.
As shown in FIG. 2, the culture device 10 comprises a culture tank 11, a raw material gas tank 12, a culture solution tank 13, an adjustment gas tank 14, a culture target tank 15, and a processor 17. The culture tank 11 accommodates a culture solution 18. Examples of the hydrogen-oxidizing bacteria 21 include bacteria described in JP6528295B, such as a bacterium of the genus Hydrogenophilus. In this case, as described in JP6528295B, the culture solution 18 is, for example, an aqueous solution obtained by dissolving ammonium sulfate $((NH_4)_2SO_4)$, potassium dihydrogen phosphate $(KH_2PO_4)$, dipotassium hydrogen phosphate $(K_2HPO_4)$, sodium chloride (NaCl), or the like in water. The culture tank 11 is an example of a "culture tank" according to the technology of the present disclosure. In addition to or instead of the raw material gas tank 12, a gas generation device that generates the raw material gas 26 may be provided.

Each of supply paths 12A, 13A, 14A, and 15A of each of the raw material gas tank 12, the culture solution tank 13, the adjustment gas tank 14, and the culture target tank 15 is connected to the culture tank 11. Each of the supply paths 12A, 13A, 14A, and 15A is composed of a pipe, a valve, and the like. In addition, a flowmeter 23 that measures a flow rate of a fluid flowing through the pump 22 and each supply path is disposed in each of the supply paths 12A, 13A, 14A, and 15A.

The culture solution 18 is accommodated in the culture solution tank 13. The culture solution 18 is supplied from the culture solution tank 13 to the culture tank 11 through the supply path 13A by driving the pump 22 on the supply path 13A. A supply timing and a supply amount of the culture solution 18 are controlled by the processor 17 that controls the driving of the pump 22.

(Pretreatment for Culture Solution in Culture Solution Tank)

In addition, as an example, a deaerator 13B is provided in the culture solution tank 13. In a case where the culture solution 18 is replenished from the culture solution tank 13 to the culture tank 11, the deaerator 13B removes an unnecessary gas dissolved in the culture solution 18. The unnecessary gas is, for example, a gas that adversely affects culture (reduces culture efficiency or the like), such as air and nitrogen.

The culture solution tank 13 and the raw material gas tank 12 may be connected by a pipe or the like so that the raw material gas can be replenished to the culture solution 18 before being supplied to the culture tank 11. By replenishing the raw material gas in advance to the culture solution 18 before being supplied to the culture tank 11, the dissolved gas concentration of the raw material gas in the culture solution 18 supplied to the culture tank 11 can be improved. In this manner, the culture efficiency may be improved. In this case, a dissolved gas concentration measurement unit (similar to a dissolved gas concentration measurement unit 49 described later) that measures the dissolved gas concentration of the raw material gas of the culture solution 18 may be provided in the culture solution tank 13, and a replenishment amount of the raw material gas may be controlled according to the measured dissolved gas concentration.

The culture target tank 15 accommodates the hydrogen-oxidizing bacteria 21 which are an example of the culture target. The hydrogen-oxidizing bacteria 21 are supplied from the culture target tank 15 to the culture tank 11 through the supply path 15A by driving the pump 22 on the supply path 15A. A supply timing and a supply amount of the hydrogen-oxidizing bacteria 21 are controlled by the processor 17 that controls the driving of the pump 22.

A raw material gas 26 is accommodated in the raw material gas tank 12. As described above, the raw material gas 26 is, for example, a gas containing each component such as hydrogen $(H_2)$, oxygen $(O_2)$, and carbon dioxide $(CO_2)$. The raw material gas 26 is supplied from the raw material gas tank 12 to the culture tank 11 through the supply path 12A by driving the pump 22 on the supply path 12A. A supply timing and a supply amount of the raw material gas 26 are controlled by the processor 17 that controls the driving of the pump 22.

A downstream end of the supply path 12A is disposed in the culture tank 11, and a sparger 28 is provided at the downstream end. The sparger 28 is provided for dispersing and supplying the raw material gas 26 into the culture solution 18. As the sparger 28, for example, a ventilation pipe formed of a porous material is used. The sparger 28 is an example of a bubble generation device that generates the raw material gas 26 as bubbles in the culture solution 18. The bubble generation device includes various types such as a microporous type such as the sparger 28, a swirling-flow type that generates bubbles by a high-speed swirling flow, an ejector type (also referred to as a Venturi type) that generates bubbles by a rapid pressure change in a gas-liquid flow path, and a static mixer type that generates bubbles by a shearing force of an obstacle in a gas-liquid flow path. As the bubble generation device, a type other than the sparger 28 may be used.

A part of the raw material gas 26 supplied into the culture solution 18 is dissolved in the culture solution 18. The dissolved raw material gas 26 is taken in by the hydrogen-oxidizing bacteria 21 and is consumed for the proliferation of the hydrogen-oxidizing bacteria 21 or the production of the organic substance 29. The organic substance 29 is, for example, alcohol or an amino acid. The raw material gas 26 that is not dissolved in the culture solution 18 is released into a space 11A present above a liquid surface 18A of the culture solution 18. The space 11A is filled with a mixed gas 31 which is the raw material gas 26 released without being dissolved in the culture solution 18. The raw material gas tank 12 and the pump 22 on the supply path 12A are examples of a "raw material gas supply unit".

An adjustment gas 33 is accommodated in the adjustment gas tank 14. The adjustment gas 33 is supplied from the adjustment gas tank 14 to the space 11A of the culture tank 11 through the supply path 14A by driving the pump 22 on the supply path 14A. A supply timing and a supply amount of the adjustment gas 33 are controlled by the processor 17 that controls the driving of the pump 22. The mixed gas 31 in the space 11A includes hydrogen and oxygen, which are components of the raw material gas 26. The hydrogen is a combustible gas. As will be described later, the adjustment gas 33 is used to maintain the composition ratio of the mixed gas 31 in the space 11A at a target value outside a preset explosive range.

In addition, the mixed gas 31 includes the components of the raw material gas 26. Therefore, a part of the mixed gas 31 can be returned to the culture tank 11 via a gas circulation path 39 and reused as the raw material gas 26. The mixed gas 31 to be reused as the raw material gas 26 is also supplied to the culture tank 11 via the sparger 28. The mixed gas 31 that is not reused is discharged. Although not shown, in a case where the mixed gas 31 that is not reused is discharged, the mixed gas 31 is diluted with an inert gas such as carbon dioxide and then released into the atmosphere.

A discharge path 36 for taking out the mixed gas 31 is connected to the culture tank 11, and a pump 22 and a three-way valve 38 are provided on the discharge path 36. The pump 22 generates a pressure for discharging and circulating the mixed gas 31. The three-way valve 38 switches between a state in which the discharge path 36 communicates with the gas circulation path 39 and a state in which the communication between the discharge path 36 and the gas circulation path 39 is closed. The three-way valve 38 switches whether the mixed gas 31 is sent to the gas circulation path 39 or is discharged to the outside. In addition, the discharge path 36 is provided with a composition ratio measurement unit 16 that measures the composition ratio of the mixed gas 31. The composition ratio measurement unit 16 is used for controlling the composition ratio of the mixed gas 31 described later.

(Position of Mixed Gas Extraction Port)

In addition, a mixed gas extraction port 34 for taking out the mixed gas 31 from the culture tank 11 is disposed at a position where the foam layer 18B formed on the liquid surface 18A of the culture solution 18 in the culture tank 11 can be taken in. The foam layer 18B functions as a liquid film covering the liquid surface 18A, and also contributes to suppressing the explosion by increasing a heat capacity of the space 11A. However, on the other hand, the foam layer 18B may also damage the hydrogen-oxidizing bacteria 21 and the like in addition to adversely affecting various sensors disposed in the culture tank 11. Therefore, by disposing the mixed gas extraction port 34 at a position where the foam layer 18B can be taken in, it is possible to recover a part of the foam layer 18B in a case where the mixed gas 31 is taken out, and it is possible to reduce the adverse effect caused by the foam layer 18B.

(Supply of Each Component of Raw Material Gas)

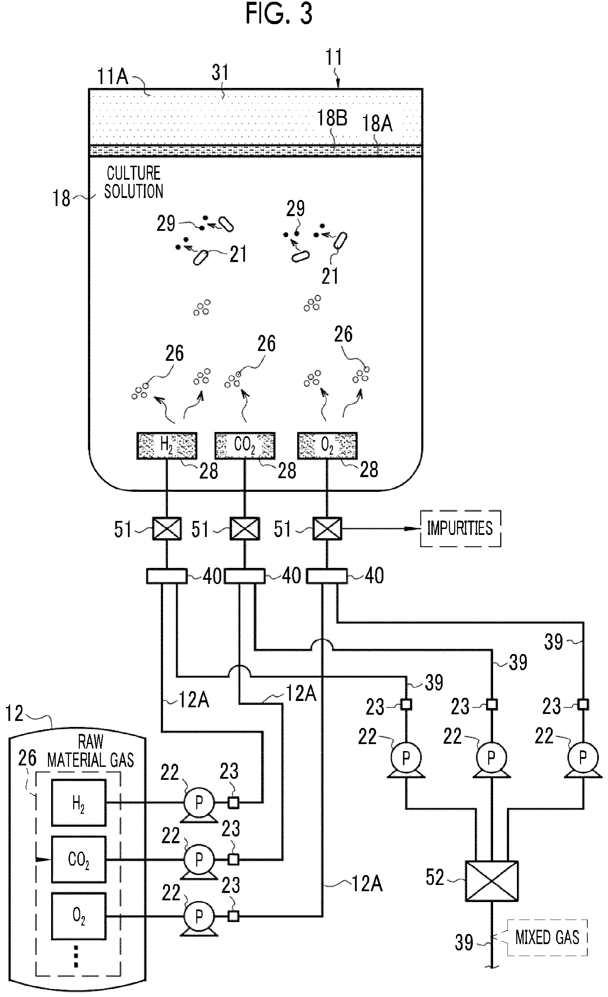

In addition, in FIG. 2, for convenience of simplifying the drawing, the supply path 12A of the raw material gas 26 and the gas circulation path 39 of the mixed gas 31 are shown as one. However, in reality, as shown in FIG. 3, the supply path 12A and the gas circulation path 39 are separated for each component such as hydrogen, oxygen, and carbon dioxide, and a plurality of components can be individually supplied. A pump 22 and a flowmeter 23 are provided in each supply path 12A and gas circulation path 39 for each component. In addition, the sparger 28 is also provided for each component such as hydrogen ($H_2$), oxygen ($O_2$), and carbon dioxide ($CO_2$). Each supply path 12A and gas circulation path 39 for each component are connected to each sparger 28 corresponding to each component.

(Removal of Impurities from Raw Material Gas)

A connecting member 40 connects the supply path 12A and the gas circulation path 39 to each other on an upstream side of the sparger 28 for each component. As a result, the supply path 12A and the gas circulation path 39 are integrated into one and are connected to the sparger 28. An impurity removal mechanism 51 is provided between each connecting member 40 and each sparger 28. The impurity removal mechanism 51 removes impurities from the raw material gas 26 supplied to the culture tank 11. The impurities include sulfur oxide or nitrogen oxide (NO, $NO_2$, and the like). The impurity removal mechanism 51 is a mechanism that uses any of a membrane separation method, a cryogenic separation method, a physical adsorption method, or a chemical absorption method, as a method of separating impurities from the raw material gas 26.

In FIG. 2, the impurities accumulate in the culture tank 11 in a case where the culture process is continued. As will be described later, a part of the culture solution 18 in the culture tank 11 is taken out once and returned to the culture tank 11 to be reused. In a case where the culture solution 18 circulates, the raw material gas 26 is separated from the culture solution 18, and the raw material gas 26 in the culture solution 18 is also returned to the culture tank 11 via a gas reuse path 62 to be reused. In addition, a part of the mixed gas 31 is also returned to the culture tank 11 via the gas circulation path 39 to be reused. The gas reuse path 62 and the gas circulation path 39 are connected to an upstream side of the impurity removal mechanism 51. The mixed gas 31 to be reused and a separation gas separated from the culture solution 18 are resupplied to the culture tank 11 after the impurities are removed by the impurity removal mechanism 51. Since the impurities are removed while circulating the raw material gas 26 in this way, accumulation of the impurities in the culture tank 11 can be suppressed.

(Separation of Components Contained in Mixed Gas)

In addition, since the components such as hydrogen, oxygen, and carbon dioxide are mixed in the mixed gas 31, the gas circulation path 39 is provided with a component separation mechanism 52 that separates each component contained in the mixed gas 31. Each component contained in the mixed gas 31 is separated by the component separation mechanism 52, and each separated component is supplied to each gas circulation path 39 provided for each component. The component separation mechanism 52 is a mechanism that uses any of a membrane separation method, a cryogenic separation method, a physical adsorption method, or a chemical absorption method, as a method of separating each component from the mixed gas 31. The component separation mechanism 52 is an example of a "first component separation mechanism" according to the technology of the present disclosure.

As described above, in a case where the raw material gas 26 is supplied to the culture tank 11, there are advantages in terms of culture efficiency and safety by individually supplying each component such as hydrogen ($H_2$), oxygen ($O_2$), and carbon dioxide ($CO_2$). The advantage of the culture efficiency is that the raw material gas 26 supplied to the culture tank 11 is easily adjusted to an appropriate composition from the viewpoint of the culture efficiency. In addition, the advantages of the safety are as follows. That is, three elements of explosion are a combustible gas, oxygen, and an ignition source, but before the raw material gas 26 is supplied to the culture tank 11, hydrogen, which is a combustible gas, and oxygen among the three elements of explosion can be separated and handled, and a risk of explosion can be further reduced.

The component separation mechanism 52 may also separate components such as nitrogen and methane as the components contained in the mixed gas 31, in addition to hydrogen, oxygen, and carbon dioxide. Since nitrogen, methane, and the like have an action of reducing the solubility of the raw material gas 26 in the culture solution 18, in a case where the mixed gas 31 is reused, the solubility of the raw material gas 26 can be improved by separating these gases.

(Circulation, Recovery, and Reuse of Culture Solution)

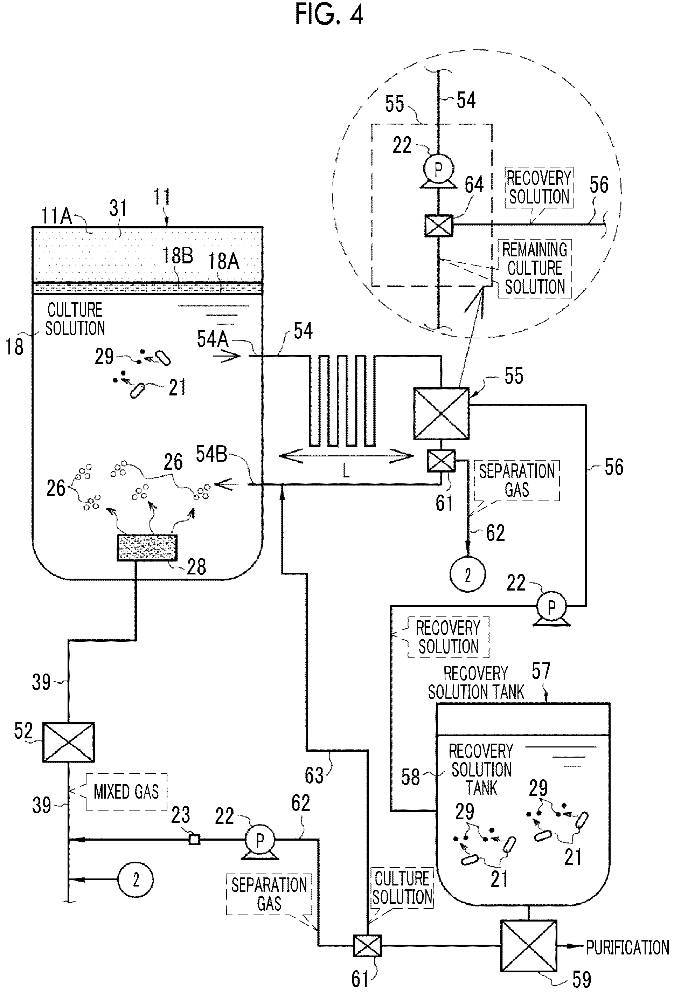

As shown in FIG. 4 in addition to FIG. 2, a culture solution circulation path 54 is connected to the culture tank 11, and a culture solution recovery mechanism 55 is provided in the culture solution circulation path 54. The culture solution circulation path 54 takes out a part of the culture solution 18 from the culture tank 11. The culture solution recovery mechanism 55 recovers the culture solution 18 including the organic substance 29 from the culture solution 18 taken out from the culture tank 11 through the culture solution circulation path 54. The culture solution circulation path 54 returns the culture solution 18 containing the hydrogen-oxidizing bacteria 21, which is a remaining culture solution 18 after being recovered by the culture solution recovery mechanism 55, to the culture tank 11. It should be noted that, in a case of purifying the proliferated hydrogen-oxidizing bacteria 21, the hydrogen-oxidizing bacteria 21 is recovered by a method different from the organic substance 29.

(Separation of Dissolved Gas Contained in Culture Solution)

In the culture solution circulation path 54, a gas separation mechanism 61 is provided on a downstream side of the culture solution recovery mechanism 55. The gas separation mechanism 61 separates a gas containing the components of the raw material gas 26 contained in the culture solution 18 from the culture solution 18 as a separation gas before returning the culture solution 18 to the culture tank 11. Then, the culture solution 18 from which the separation gas has been separated is returned to the culture tank 11. The gas separation mechanism 61 is a separation mechanism that uses any of a membrane separation method, a cryogenic separation method, a physical adsorption method, or a chemical absorption method. A hydrogen storage alloy may be used for the separation of hydrogen.

(Reuse of Raw Material Gas from Culture Solution)

A part of the separation gas separated by the gas separation mechanism 61 is returned to the culture tank 11 via the gas reuse path 62 and the gas circulation path 39 of the mixed gas 31. Then, the part of the separation gas is reused as the raw material gas 26. Therefore, use efficiency of the raw material gas 26 is improved.

(Explosion Suppression in Case of Reusing Raw Material Gas from Culture Solution)

In addition, since the separation gas is returned to the culture tank 11 via the gas circulation path 39, the separation gas is separated into respective components such as hydrogen, oxygen, and carbon dioxide by the component separation mechanism 52 as described above. However, the separation gas may be separated into respective components such as hydrogen, oxygen, and carbon dioxide in the gas reuse path 62 instead of being separated for each component in the component separation mechanism 52. Each of these separated components is reused via the gas circulation path 39 provided for each component. The separation of the separation gas in the gas reuse path 62 into each component may be performed, for example, by the gas separation mechanism 61 that separates the gas from the culture solution 18.

In this way, in a case where the separation gas is reused, hydrogen, which is a combustible gas, and oxygen can be separated, so that the safety in the gas reuse path 62 is improved. As described above, from the viewpoint of safety, it is preferable to separate hydrogen, which is a combustible gas, from the culture solution 18 taken out from the culture tank 11 as upstream as possible. As for the order of separating the components, it is preferable to separate hydrogen first and then separate oxygen and carbon dioxide. Such a gas separation mechanism 61 has a function of suppressing explosion, and is therefore an example of an "explosion suppression mechanism" according to the technology of the present disclosure.

In addition, in a case where the respective components of the separation gas are separated in the gas separation mechanism 61 in this way, the carbon dioxide can be reused as the raw material gas 26 and can also be returned to the adjustment gas tank 14 to be reused as the adjustment gas 33. The gas separation mechanism 61 is also an example of a "second component separation mechanism" according to the technology of the present disclosure in a case of separating the separation gas into each component. Also in the gas separation mechanism 61, as in the component separation mechanism 52, components that reduce the solubility of the raw material gas 26, such as nitrogen and methane, may also be separated in addition to hydrogen, oxygen, and carbon dioxide.

(Separation of Specific Component from Culture Solution)

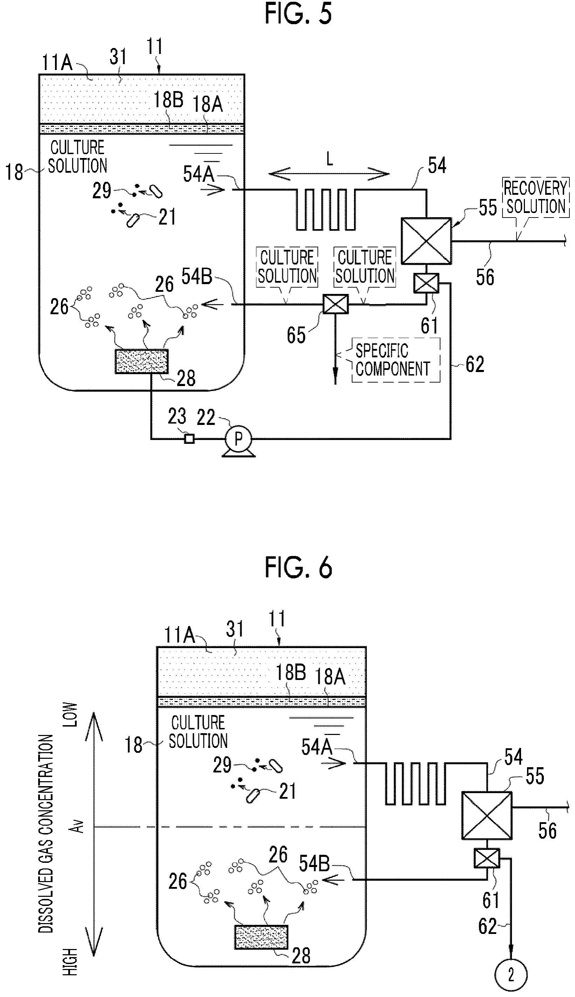

In addition, as shown in FIG. 5, by using a separation mechanism 65 similar to the gas separation mechanism 61 and the component separation mechanism 52, in addition to separating the gas from the culture solution 18, a specific component contained in the culture solution 18 may be separated. Examples of the specific component include ammonia and an ammonium compound. The ammonia and the ammonium compound serve as a nutrient source for the hydrogen-oxidizing bacteria 21. By separating these components, the separated specific component can be reused while adjusting the supply amount.

In this way, by separating various components contained in the mixed gas 31 and the culture solution 18, it is possible to remove unnecessary components and reuse those that can be reused, and it is possible to improve the culture efficiency and the use efficiency of the raw material gas 26.

In FIG. 4, the culture solution recovery mechanism 55 is connected to a recovery solution tank 57 that stores a recovery solution 58 which is the recovered culture solution 18, via a recovery path 56. The recovery solution 58 is transferred from the culture solution recovery mechanism 55 to the recovery solution tank 57 through the recovery path 56. A pump 22 for transferring the recovery solution 58 is provided in the recovery path 56.

A part of the recovery solution 58 accommodated in the recovery solution tank 57 is transferred to a purification device (not shown). In the purification device, the organic substance 29 produced by culturing the hydrogen-oxidizing bacteria 21 is extracted from the recovery solution 58.

(Process of Reducing Dissolved Combustible Gas Concentration in Recovery Solution)

The culture device 10 comprises a concentration reduction mechanism that reduces a dissolved hydrogen concentration in the recovery solution 58 to be lower than a dissolved hydrogen concentration in the culture solution 18 in the culture tank 11. The concentration reduction mechanism is configured, for example, by extending a total length L of a section from the culture tank 11 to the culture solution recovery mechanism 55 in the culture solution circulation path 54 to a length required for reducing the concentration. Specifically, the total length L of the section has a length required to reduce the dissolved hydrogen concentration in the recovery solution 58 to 1/10 or less of the dissolved hydrogen concentration in the culture solution 18 in the culture tank 11. The culture solution 18 taken out from the culture tank 11 contains the hydrogen-oxidizing bacteria 21 that consume hydrogen, and the hydrogen-oxidizing bacteria 21 also consume hydrogen in the culture solution circulation path 54. As the culture solution circulation path 54 is longer, a time for which the hydrogen is consumed by the hydrogen-oxidizing bacteria 21 is ensured. In this manner, use efficiency of hydrogen is improved. In addition, in a case where hydrogen is consumed in a section of the culture solution circulation path 54 up to the culture solution recovery mechanism 55, the dissolved hydrogen concentration of the recovery solution 58 recovered by the culture solution recovery mechanism 55 is reduced. As a result, the hydrogen released from the recovery solution 58 is also reduced, so that the risk of explosion in the recovery solution tank 57 is also reduced, and the safety is ensured. The culture solution circulation path 54 of which the total length L of the above-described section has a length required for reducing the concentration is an example of a "concentration reduction mechanism" according to the technology of the present disclosure.

In addition, instead of extending the total length L of the section of the culture solution circulation path 54, a flow rate of the culture solution 18 flowing through the section may be decreased. As a result, since a time until the culture solution 18 taken out from the culture tank 11 is transferred to the culture solution recovery mechanism 55 is extended, a time for which the hydrogen is consumed in the taken-out culture solution 18 can be extended. As a result, the same effect as in a case where the total length L of the section is extended can be obtained. The flow rate of the culture solution 18 can be adjusted, for example, by controlling a suction amount of the pump 22 per unit time. The suction amount of the pump 22 is controlled by the processor 17. A flow rate adjustment mechanism configured by the processor 17 and the pump 22 is also an example of a "concentration reduction mechanism" according to the technology of the present disclosure.

(Downstream Side of Recovery Solution Tank 57)

On a downstream side of the recovery solution tank 57, a purification target separation mechanism 59 is provided to separate a part of the recovery solution 58, which contains the organic substance 29, from the recovery solution 58 as a purification target and send the separated purification target to the purification device. The remaining recovery solution 58 other than the recovery solution 58 separated as the purification target by the purification target separation mechanism 59 is returned to the culture tank 11 as the culture solution 18 via a liquid reuse path 63 and the culture solution circulation path 54 and reused.

As shown in an enlarged view of a part of FIG. 4, the culture solution recovery mechanism 55 comprises the pump 22 and a liquid separation mechanism 64. The pump 22 generates a circulation pressure for circulating the culture solution 18. Similar to the purification target separation mechanism 59, the liquid separation mechanism 64 separates a part of the culture solution 18, which includes the organic substance 29, from the culture solution 18 taken out from the culture tank 11, and sends the separated culture solution 18 to the recovery path 56 as the recovery solution 58. The remaining culture solution 18 is sent to the gas separation mechanism 61.

(Position of Outlet of Culture Solution in Circulation Path of Culture Solution)

In addition, as shown in FIG. 6, the culture solution circulation path 54 has an outlet 54A for taking out the culture solution 18 from the culture tank 11, and a return port 54B for returning a part of the taken-out culture solution 18 to the culture tank 11. In the culture solution 18 in the culture tank 11, the dissolved gas concentration of the raw material gas 26 is low on an upper side in a height direction, and the dissolved gas concentration is high on a lower side. The outlet 54A is set in a low-concentration region in which the dissolved gas concentration is relatively low, and the return port 54B is set in a high-concentration region in which the dissolved gas concentration is relatively high. More specifically, the outlet 54A is set in a low-concentration region in which the dissolved gas concentration of the culture solution 18 in the culture tank 11 is lower than an average value Av.

As a result, in a case where a part of the culture solution 18 is taken out from the culture tank 11 in order to circulate the culture solution 18, it is easy to eliminate a concentration unevenness of the dissolved gas concentration of the culture solution 18 in the culture tank 11.

(Stirring of Culture Tank, Temperature Adjustment, and Sensing of Various Types of Information)

In addition, in FIG. 2, the stirring rod 41 that stirs the culture solution 18 is provided in the culture tank 11. The stirring rod 41 is a support rod provided with a screw 41A, and is provided such that the screw 41A is disposed in the culture solution 18. The stirring rod 41 rotates with the support rod as a rotation shaft by driving a motor 42 controlled by the processor 17. As a result, the culture solution 18 is stirred. The stirring rod 41 stirs the culture solution 18 to diffuse the hydrogen-oxidizing bacteria 21 and the raw material gas 26 in the culture solution 18 such that local uneven distribution does not occur.

In addition, the stirring rod 41 stirs the culture solution 18 to generate bubbles from the culture solution 18 and form the above-described foam layer 18B on the liquid surface 18A of the culture solution 18.

In addition, the culture tank 11 is provided with the thermometer 46, the temperature adjuster 47, the pressure gauge 48, the dissolved gas concentration measurement unit 49, and a density measurement unit 50. The thermometer 46 measures the temperature T in the culture solution 18. The temperature adjuster 47 heats or cools the culture solution 18 in the culture tank 11. The pressure gauge 48 measures the pressure PS in the space 11A. The dissolved gas concentration measurement unit 49 measures the dissolved gas concentration Dr of the raw material gas 26 dissolved in the culture solution 18. The density measurement unit 50 measures a density $\rho$ of the hydrogen-oxidizing bacteria 21 present in the culture solution 18 in the culture tank 11.

The culture solution 18 is maintained at a target temperature suitable for culturing the hydrogen-oxidizing bacteria 21. The target temperature is, for example, 40°C or higher and 95°C or lower. The target temperature is set for each microorganism. In a case where the hydrogen-oxidizing bacteria 21 belongs to the genus Hydrogenophilus, the target temperature is more preferably about 50°C. The culture solution 18 is heated to the target temperature, and in some cases, cooling is required. This is because the culture solution 18 may exceed the target temperature due to heat generation during the proliferation of the microorganisms to be cultured. The processor 17 controls driving of the temperature adjuster 47 such that the temperature T of the culture solution 18 is maintained at the target temperature, based on the temperature T measured by the thermometer 46.

In addition, the processor 17 controls a pressure of the mixed gas 31 in the space 11A based on the pressure PS measured by the pressure gauge 48. By setting the pressure PS of the mixed gas 31 to a target pressure, solubility of the raw material gas 26 in the culture solution 18 is improved. In this way, the processor 17 controls the pressure of the mixed gas 31 to ensure appropriate solubility in the culture solution 18. As a result, culture efficiency of the hydrogen-oxidizing bacteria 21 is improved. An adjustment target of the pressure PS of the mixed gas 31 is at least one of the supply amount of the raw material gas 26, the discharge amount of the mixed gas 31, or the supply amount of the adjustment gas 33. The processor 17 controls the driving of each pump 22 to adjust the supply amount or the discharge amount of the gas.

More specifically, the dissolved gas concentration measurement unit 49 measures the concentration of each component (hydrogen, oxygen, and carbon dioxide) contained in the raw material gas 26 dissolved in the culture solution 18. As an example, the dissolved gas concentration measurement unit 49 is composed of a dissolved hydrogen meter (for example, model number KM2100DH manufactured by Kyoei Denshi Kenkyusho) that measures a dissolved hydrogen concentration ($Dr(H_2)$), a dissolved oxygen meter (for example, model number InPro6860i manufactured by Mettle Toledo) that measures a dissolved oxygen concentration ($Dr(O_2)$), and a dissolved carbon dioxide meter (for example, model number InPro5000i manufactured by Mettle Toledo) that measures a dissolved carbon dioxide concentration ($Dr(CO_2)$). The dissolved gas concentration measurement unit 49 outputs measured values to the processor 17.

As shown in FIG. 3, the raw material gas tank 12 accommodates each component (hydrogen, oxygen, and carbon dioxide) of the raw material gas 26. In addition, the supply path 12A and the pump 22 are provided for each component.

The processor 17 acquires a measured value of the dissolved gas concentration for each component from the dissolved gas concentration measurement unit 49, compares the dissolved gas concentration with the target value for each component, and supplies the component that is deficient in the raw material gas 26 to the culture tank 11. Through such control of a supply amount of each component of the raw material gas 26, the processor 17 can maintain the dissolved gas concentration of the raw material gas 26 in the culture solution 18 at an appropriate value.

The density measurement unit 50 measures a density ρ of a culture target present in the culture solution 18 in the culture tank 11. The density measurement unit 50 is composed of, for example, a total cell density sensor (for example, model number: Dencytee RS485 manufactured by Hamilton). In the present example, since the culture target is the hydrogen-oxidizing bacteria 21, the density measurement unit 50 measures the density ρ of the hydrogen-oxidizing bacteria 21 in the culture solution 18. The density ρ of the hydrogen-oxidizing bacteria 21 has a positive correlation with a consumption amount of the raw material gas 26 consumed by the hydrogen-oxidizing bacteria 21. Measuring the density ρ can be used, for example, to calculate the consumption amount of the raw material gas 26 consumed by the hydrogen-oxidizing bacteria 21, or for a process check to verify whether or not the consumption amount of the raw material gas 26 is appropriate with respect to the amount of the hydrogen-oxidizing bacteria 21.

A water level gauge 66 measures a water level of the culture solution 18 in the culture tank 11, that is, a height of the liquid surface 18A. An amount of the culture solution 18 present in the culture tank 11 can be ascertained by the water level of the culture solution 18 measured by the water level gauge 66. The water level gauge 66 is used for a process check of whether or not the amount of the culture solution 18 is appropriate, calculation of a necessary replenishment amount of the culture solution 18, and the like.

In addition, the culture tank 11 is provided with an explosion vent 67. The explosion vent 67 is configured to be destroyed earlier than other portions in a case where an explosion occurs in the culture tank 11, thereby safely releasing the pressure inside the culture tank 11 and preventing the damage from spreading. A place where large damage does not occur even in a case where the pressure is released, or a place where measures are taken so that damage does not spread is selected as the vicinity of the explosion vent 67.

The processor 17 is configured of, for example, a central processing unit (CPU) and a random access memory (RAM), and controls each unit of the culture device 10 as described above. In addition, the memory 17A is a non-volatile memory in which various types of setting information are stored, such as a flash memory.

(Control of Bubble Diameter of Raw Material Gas Supplied to Culture Tank)

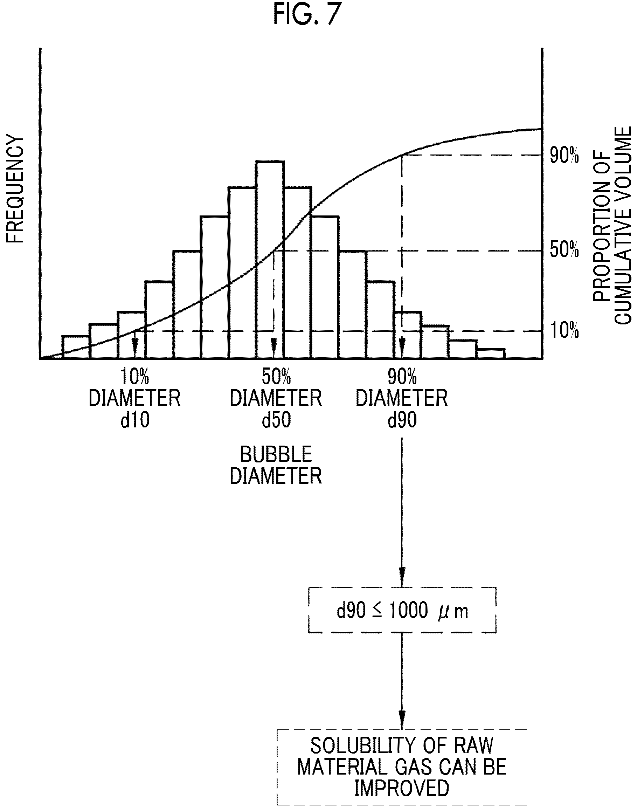

For a bubble diameter of the raw material gas 26 generated in the culture solution 18 of the culture tank 11, d90 is set to 1000 μm or less. As shown in FIG. 7, d90 refers to a bubble diameter at which a cumulative volume of bubbles is 90% of a total volume of all the bubbles in a case where volumes of all the bubbles are sequentially integrated from a bubble with the smallest diameter. A triangular distribution in a bar graph shown in FIG. 7 is a histogram of bubble diameters of the bubbles generated in the culture solution 18, in which a horizontal axis is a bubble diameter and a vertical axis is a frequency. In a case where the volumes of such bubble diameters are cumulated, a line graph sloping upward to the right as shown in FIG. 7 is obtained. d90 is a bubble diameter at which a proportion of the cumulative volume is 90% of the total volume, and in the culture device 10, for example, this bubble diameter is set to be 1000 μm or less. Such a bubble diameter is determined by, for example, specifications of a bubble generation device such as the sparger 28. Regarding the bubble diameter, by setting d90 to 1000 $\mu$m or less, the solubility of the raw material gas 26 in the culture solution 18 can be improved. A more preferable bubble diameter is that d90 is 500 $\mu$m or less, and it is still more preferable that d90 is 200 $\mu$m or less. By setting the bubble diameter to such a value, it is possible to ensure high solubility of the raw material gas 26 even under conditions in which the raw material gas 26 is difficult to dissolve, for example, in a case where the temperature of the culture solution 18 is high or in a case where a depth of the culture tank 11 is shallow.

(Surface Tension Reducer)

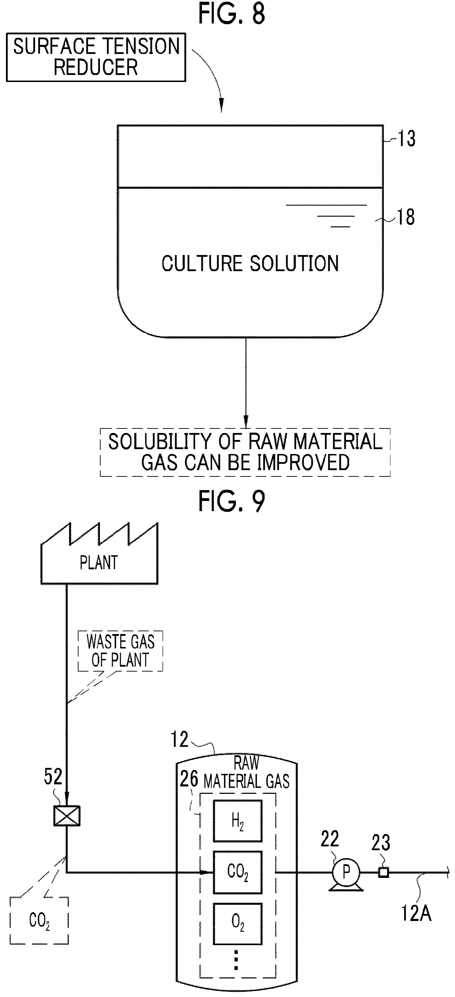

In addition, as shown in FIG. 8, the culture solution 18 may contain a surface tension reducer by adding the surface tension reducer. In a case where a surface tension of the culture solution 18 is reduced, the diameter of the bubble is likely to be reduced, and thus the solubility of the raw material gas 26 containing hydrogen, which is a combustible gas, can be improved. The surface tension of the culture solution 18 is preferably 65 mN/m or less. With such a surface tension, it is easy to set d90 of the bubble diameter to 1000 $\mu$m or less. An addition amount of the surface tension reducer is determined such that the surface tension is set to this value. The surface tension of the culture solution 18 is more preferably 55 mN/m or less and still more preferably 45 mN/m or less. By setting the surface tension to such a value, it is easy to reduce the bubble diameter.

(Reuse of Waste Gas)

In addition, as shown in FIG. 9, the culture device 10 may reuse a waste gas containing carbon dioxide discharged from an external device as the raw material gas 26 of the culture device 10. The external device is a device other than the culture device 10, and may be, for example, another device in the same facility as a facility in which the culture device 10 operates, or another device in another facility (factory or the like). In a case where the waste gas is reused, the component separation mechanism 52 separates carbon dioxide from the waste gas, and the separated carbon dioxide is reused as the raw material gas 26. In this way, by reusing carbon dioxide contained in the waste gas, it is possible to contribute to the reduction of the carbon dioxide emissions.

2. First Embodiment

The first embodiment of the culture device 10 will be described with reference to FIGS. 10 to 16 with the example of the common configuration as a premise. The culture device 10 according to the first embodiment performs composition ratio control of the mixed gas 31, dissolution rate control of the hydrogen, and consumption rate control of the hydrogen as a safety measure in a case of using the hydrogen, which is a combustible gas.

As shown in FIG. 10, the processor 17 receives a measurement signal and a detection signal from various sensors of the culture device 10. Then, the memory 17A of the processor 17 stores a target value of various controls as the culture condition information. The processor 17 performs various controls by outputting a control signal corresponding to the target value to each unit.

The target values of various controls include a target temperature and a target pressure of the culture solution 18. In addition, examples of the target value outside the explosive range used for the composition ratio control include a target value ($TD(H_2)$) of the hydrogen concentration, a target value ($TD(O_2)$) of the oxygen concentration, and a water vapor concentration (not shown). In addition, the memory 17A is set with a target value ($TRd(H_2)$) of the hydrogen dissolution rate used for the hydrogen dissolution rate control, a target value ($TRc(H_2)$) of the hydrogen consumption rate used for the hydrogen consumption rate control, and the like.

(Composition Ratio Control of Mixed Gas)

As described above, the hydrogen is a combustible gas, and the adjustment gas 33 is used to maintain the composition ratio of the mixed gas 31 in the space 11A at the target value outside the preset explosive range. In the mixed gas 31, the hydrogen, which is a combustible gas, is mixed with the oxygen. In this case, an explosion may occur depending on the composition ratio of the mixed gas 31. The composition ratio of the mixed gas in a case where the explosion occurs is referred to as an explosive range. As described above, the target value ($TD(H_2)$) of the hydrogen concentration, the target value ($TD(O_2)$) of the oxygen concentration, the water vapor concentration, and the like are set as the target value outside the explosive range in the memory 17A of the processor 17. The target value outside the explosive range varies depending on components of the mixed gas 31. In a case where the mixed gas 31 is a mixed gas including hydrogen and oxygen as in the present example, the target value outside the explosive range is generally an oxygen concentration of less than 5% or a hydrogen concentration of less than 4% in the mixed gas 31.

Furthermore, the present applicant has verified through an experiment that, in a case where a water vapor concentration is 7% or more, the composition ratio of the mixed gas can be maintained outside the explosive range even in a case where the oxygen concentration is 5% or more. Therefore, in the present example, as an example, the target value outside the explosive range is set such that, in a case where the water vapor concentration is 7% or more and 90% or less, the oxygen concentration is set in a range of 5% or more and 9% or less.

The experiment was performed by the following method. As an explosion container, a stainless steel chamber having an inner diameter of 200 mm, a depth of 200 mm, and a volume of 6 L was used. The explosion container was filled with hydrogen, oxygen, carbon dioxide, and water vapor (water vapor was filled by pouring water into the explosion container and performing vacuuming up to a saturated water vapor pressure at a temperature inside the container to vaporize the water. The hydrogen, oxygen, and carbon dioxide were filled from a gas cylinder through a gas introduction pipe provided in the explosion container). After rotating a stirring fan inside the explosion container to homogenize the gas, ignition of the gas was attempted using a thin wire explosion device provided in the explosion container. The determination of ignition or non-ignition was performed based on the presence or absence of a pressure increase and the presence or absence of generation of a negative pressure inside the explosion container due to the generation of water. The pressure was measured by a pressure sensor (PHS-B-10MP manufactured by Kyowa Electronic Instruments Co., Ltd.) disposed on an upper valve of a gas discharge pipe provided in the explosion container.

Table 1 shows experimental results of whether or not ignition occurs depending on a gas concentration of the mixed gas. Both Example 1 and Comparative Example 1 in Table 1 are examples in which the water vapor concentration is 0%, and concentrations other than the oxygen concentration are almost the same. In Example 1 in which ignition was not made, the oxygen concentration was 4% which is less than 5%, and in Comparative Example 1 in which ignition was made, the oxygen concentration was 9% which exceeds 5%. As is generally known, experimental results of Example 1 and Comparative Example 1 show that, in a case where the water vapor concentration is 0%, ignition does not occur in a case where the oxygen concentration is less than 5%.

In addition, all of Example 2, Example 3, and Comparative Example 2 are examples in which the water vapor concentration is 7% or more, and only the oxygen concentration is significantly different. In Example 2 and Example 3, the oxygen concentration was 9%, and in Comparative Example 2, the oxygen concentration was 16% which exceeds 9%. From these experimental results, it was found that in a case where the water vapor concentration is 7% or more, the ignition does not occur even in a case where the oxygen concentration is 5% or more but 9% or less.

[Table 1]

| | Gas Concentration | | | | Temperature | Result |
|---|---|---|---|---|---|---|
| | $H_2$ | $O_2$ | $CO_2$ | $H_2O$ | | |
| Example 1 | 72% | 4% | 24% | 0% | 31°C | Non-ignition |
| Example 2 | 70% | 9% | 14% | 7% | 44°C | Non-ignition |
| Example 3 | 66% | 9% | 13% | 12% | 52°C | Non-ignition |
| Comparative Example 1 | 67% | 9% | 24% | 0% | 31°C | Ignition |
| Comparative Example 2 | 59% | 16% | 13% | 12% | 52°C | Ignition |

In Table 1, there are no experimental results in a case where the hydrogen concentration is less than 4%, but it is known that in a case where the hydrogen concentration is less than 4% instead of the oxygen concentration being less than 5%, the mixed gas will not ignite even in a case where the water vapor concentration is 0%. Therefore, as the target value outside the explosive range, the hydrogen concentration of the mixed gas 31 may be less than 4% instead of the oxygen concentration of the mixed gas 31 being less than 5%.

The adjustment gas 33 is, for example, an inert gas. More specifically, in the present example, carbon dioxide ($CO_2$) is used. By supplying the adjustment gas 33 to the space 11A, the oxygen concentration or the hydrogen concentration decreases, and the composition ratio of the mixed gas 31 is maintained at the target value outside the explosive range. In addition to the inert gas, any gas can be used as the adjustment gas 33. For example, any gas may be used as the adjustment gas 33, such as hydrogen, which is a combustible gas, to set the oxygen concentration of the mixed gas 31 to less than 5%, so that the composition ratio of the mixed gas 31 is set to the target value outside the explosive range. As described above, any gas can be used as the adjustment gas 33, but as will be described later, in a case where the mixed gas 31 is reused, it is preferable to use the components of the raw material gas 26 as the adjustment gas 33.

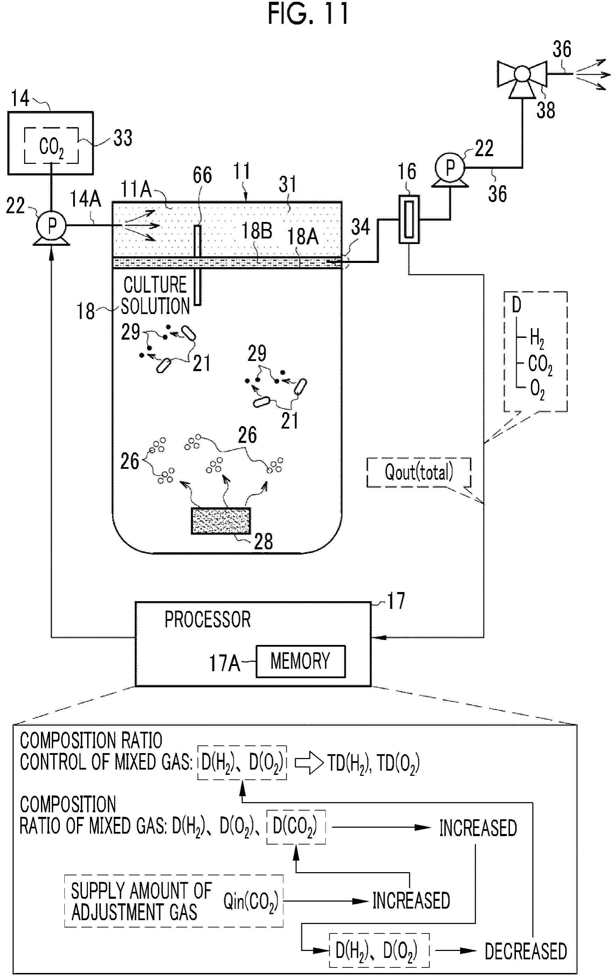

As shown in FIG. 11, the composition ratio measurement unit 16 is disposed on the discharge path 36 for discharging the mixed gas 31 from the space 11A of the culture tank 11. The composition ratio measurement unit 16 includes, for example, a gas analyzer (for example, manufactured by HORIBA, Ltd.: model number TCA-51d) that measures a hydrogen concentration ($D(H_2)$), a gas analyzer (for example, manufactured by HORIBA, Ltd.: model number VA-5113) that measures an oxygen concentration ($D(O_2)$), a gas analyzer (for example, manufactured by HORIBA, Ltd.: model number VA-5113) that measures a carbon dioxide concentration ($D(CO_2)$), and a flowmeter (for example, manufactured by Emerson: model number CMFS007M) that measures a total flow rate ($Qout(total)$) of the mixed gas 31.

Accordingly, the composition ratio measurement unit 16 is capable of measuring the concentrations of each component of the mixed gas 31 and the total flow rate. The composition ratio measurement unit 16 outputs each measurement value to the processor 17 as a measurement signal.

The processor 17 determines a supply amount of the adjustment gas 33 such that the oxygen concentration of the mixed gas 31 is maintained below a preset upper limit value, for example, based on the measured values of the total flow rate and the oxygen concentration of the mixed gas 31. Then, the processor 17 controls the pump 22 to supply the determined supply amount of the adjustment gas 33 from the adjustment gas tank 14 to the space 11A. As a result, the supply amount ($Qin(CO_2)$) of the carbon dioxide in the space 11A is increased, the oxygen concentration ($D(O_2)$) is relatively reduced, and the composition ratio of the mixed gas 31 in the space 11A is maintained at the target value ($TD(O_2)$) outside the preset explosive range. By increasing the supply amount of the adjustment gas 33, the hydrogen concentration ($D(H_2)$) is also reduced, so that the hydrogen concentration can also be maintained at the target value ($TD(O_2)$) outside the explosive range. The processor 17, the adjustment gas tank 14, and the pump 22 on the supply path 14A are examples of a "composition ratio control mechanism" according to the technology of the present disclosure.

In addition, since both the culture solution 18 and the space 11A are present in the culture tank 11, in a case where the temperature T of the culture solution 18 is maintained at the target temperature, a temperature in the space 11A can also be maintained at substantially the same temperature. As described above, the composition ratio of the mixed gas 31 in the space 11A is maintained at the target value outside the preset explosive range. The target value also includes the water vapor concentration of the mixed gas 31. A temperature of the mixed gas 31 can be made to be 40°C or higher by controlling the temperature of the culture solution 18 to 40°C or higher. As a result, the water vapor concentration of the mixed gas 31 can also be adjusted to 7% or more.

(Dissolution Rate Control of Combustible Gas)

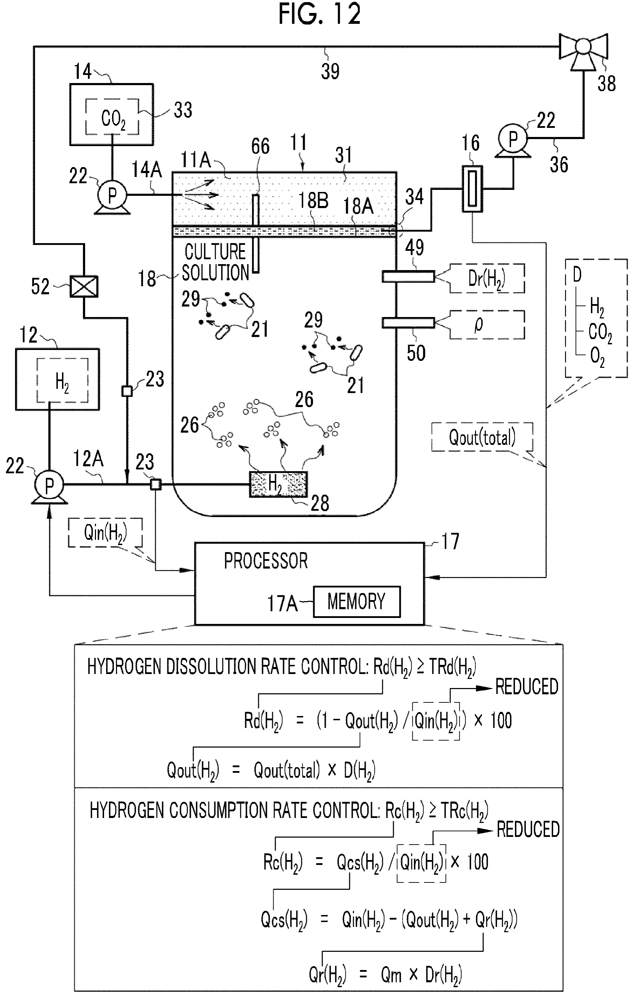

As shown in FIG. 12, the dissolution rate control mechanism including the processor 17 executes hydrogen dissolution rate control as an example of the combustible gas dissolution rate control. The processor 17, the composition ratio measurement unit 16, and the flowmeter 23 are examples of a "dissolution rate control mechanism" according to the disclosed technology.

The dissolution rate control mechanism measures, during the culture, a hydrogen dissolution rate, which is a proportion of the hydrogen dissolved in the culture solution 18, of the hydrogen, which is a combustible gas contained in the raw material gas supplied to the culture tank 11 through the gas circulation path 39 or the raw material gas supply unit, and maintains the measured hydrogen dissolution rate ($Rd(H_2)$) at or above a preset target value ($TRd(H_2)$). The hydrogen dissolution rate ($Rd(H_2)$) is defined by the following Expression (1) in a case where an amount of the hydrogen discharged from the culture tank 11 as the mixed gas 31 is defined as $Qout(H_2)$ and an amount of the hydrogen supplied to the culture tank 11 is defined as $Qin(H_2)$.

$$Rd(H_2)(\%) = (1 - Qout(H_2)/Qin(H_2)) \times 100 \ ... \ \text{Expression (1)}$$

That is, a value obtained by subtracting the amount of the hydrogen discharged from the culture tank 11 from the amount of the hydrogen supplied to the culture tank 11 is the amount of the hydrogen dissolved in the culture solution 18, and a proportion of the dissolved amount of the hydrogen to the supplied amount of the hydrogen is the hydrogen dissolution rate.

$Qout(H_2)$ is a value obtained by multiplying the total amount ($Qout(total)$) of the mixed gas 31 discharged from the culture tank 11 by the hydrogen concentration ($D(H_2)$) of the mixed gas 31. The processor 17 acquires $Qin(H_2)$ from the flowmeter 23, acquires $Qout(total)$ and the hydrogen concentration ($D(H_2)$) from the composition ratio measurement unit 16, and measures the hydrogen dissolution rate ($Rd(H_2)$) based on the acquired measurement values.

Then, the processor 17 controls, for example, the supply amount ($Qin(H_2)$) of the hydrogen to the culture tank 11 such that the hydrogen dissolution rate ($Rd(H_2)$) is maintained at or above the target value ($TRd(H_2)$). By reducing the supply amount of the hydrogen to the culture tank 11, the hydrogen dissolution rate ($Rd(H_2)$) can be increased from the definition of Expression (1). The target value of the hydrogen dissolution rate is preferably 90% or more, for example.

In a case where the hydrogen dissolution rate is increased, the amount of the hydrogen released into the space 11A is reduced. In addition to the composition ratio control mechanism of the mixed gas 31, the hydrogen concentration of the mixed gas 31 can be further reduced by performing the dissolution rate control, so that the risk of the explosion is reduced, and the safety is improved as compared with the related art. Such control is an effective technology in a case of using the raw material gas 26 containing a combustible gas such as hydrogen.

(Consumption Rate Control of Combustible Gas)

Further, as shown in FIG. 12, the dissolution rate control mechanism may control the hydrogen consumption rate through the dissolution rate control.

Specifically, the dissolution rate control mechanism measures, during the culture, a hydrogen consumption rate of the hydrogen consumed by the hydrogen-oxidizing bacteria 21, of the hydrogen, which is a combustible gas contained in the raw material gas 26 supplied to the culture tank 11 through the gas circulation path 39 or the raw material gas supply unit, and maintains the measured hydrogen consumption rate ($Rc(H_2)$) at a target value ($TRc(H_2)$) of 90% or more through control of the hydrogen dissolution rate.

The hydrogen consumption rate ($Rc(H_2)$) is defined by the following Expression (2) in a case where the amount of the hydrogen consumed by the hydrogen-oxidizing bacteria 21 in the culture tank 11 is defined as $Qcs(H_2)$.

$$Rc(H_2)(\%) = Qcs(H_2)/Qin(H_2) \times 100 \ ... \ \text{Expression (2)}$$

That is, the hydrogen consumption rate is a proportion of the amount of the hydrogen consumed by the hydrogen-oxidizing bacteria 21 in the culture tank 11 to the amount of the hydrogen supplied to the culture tank 11.

A difference between $Qin(H_2)$, which is the supply amount of the hydrogen to the culture tank 11, and $Qout(H_2)$, which is the amount of the hydrogen discharged from the culture tank 11, indicates the amount of the hydrogen dissolved in the culture solution 18. The amount of the hydrogen dissolved in the culture solution 18 includes a dissolved hydrogen amount that is dissolved in the culture solution 18 but is not consumed in addition to $Qcs(H_2)$, which is the amount consumed by the hydrogen-oxidizing bacteria 21. Therefore, in a case where the dissolved hydrogen amount is defined as $Qr(H_2)$, $Qcs(H_2) = Qin(H_2) - (Qout(H_2) + Qr(H_2))$ is established. Here, the dissolved hydrogen amount $Qr(H_2)$ refers to a change amount from a reference dissolved hydrogen amount. For example, in a case where $Qcs(H_2)$, $Qin(H_2)$, and $Qout(H_2)$ are defined as the hydrogen consumption amount, the hydrogen supply amount, and the hydrogen discharge amount per minute, respectively, the dissolved hydrogen amount $Qr(H_2)$ refers to a change amount from the dissolved hydrogen amount (the amount of the hydrogen dissolved in the culture solution 18) one minute ago.

In a case where a storage capacity of the culture solution 18 accommodated in the culture tank 11 is defined as $Qm$, the dissolved hydrogen amount ($Qr(H_2)$) is obtained by multiplying the storage capacity ($Qm$) of the culture solution 18 by the dissolved hydrogen concentration ($Dr(H_2)$). The processor 17 measures the dissolved hydrogen amount ($Qr(H_2)$) by acquiring the dissolved hydrogen concentration ($Dr(H_2)$) measured from the dissolved gas concentration measurement unit 49 and calculating the storage capacity ($Qm$) of the culture solution 18 based on the water level acquired from the water level gauge 66. Then, the processor 17 measures the hydrogen consumption amount ($Qcs(H_2)$) and the hydrogen consumption rate ($Rc(H_2)$) according to Expression (2).

The dissolution rate control mechanism including the processor 17 maintains the hydrogen consumption rate ($Rc(H_2)$) at 90% or more, which is the target value ($TRc(H_2)$), through the dissolution rate control of reducing the supply amount of the hydrogen. By improving the hydrogen consumption rate, the use efficiency of the raw material gas 26 is improved.

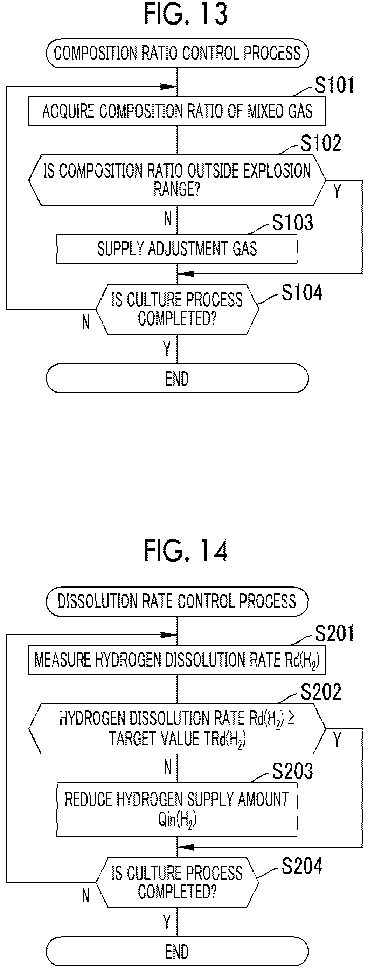

Hereinafter, an operation of the above-mentioned configuration will be described with reference to a flowchart shown in FIGS. 13 to 15. FIG. 13 shows a procedure of a composition ratio control process. For example, the processor 17 drives the temperature adjuster 47 in a state in which the culture solution 18 containing the hydrogen-oxidizing bacteria 21 is accommodated in the culture tank 11, to heat the temperature of the culture solution 18 to the target temperature. The processor 17 starts a culture process by starting the supply of the raw material gas 26 after the culture solution 18 reaches the target temperature. Even after the start of the culture process, the processor 17 controls the temperature of the culture solution 18 to the target temperature through the temperature adjuster 47. In the culture process, the hydrogen-oxidizing bacteria 21 proliferate by taking in the raw material gas 26 dissolved in the culture solution 18, and further produce the organic substance 29. The hydrogen-oxidizing bacteria 21 efficiently produce the organic substance 29 by modifying the gene. A timing at which the hydrogen-oxidizing bacteria 21 produce the organic substance 29 is during or after the proliferation. A part of the raw material gas 26 that is not dissolved in the culture solution 18 is released into the space 11A above the liquid surface 18A of the culture solution 18, and becomes the mixed gas 31.

In step S101 of the composition ratio control process, the processor 17 acquires the measured values of the concentration of each component of the mixed gas 31 and the total flow rate of the mixed gas 31 from the composition ratio measurement unit 16. Then, the processor 17 calculates the oxygen concentration of the mixed gas 31 based on the acquired measurement values, and acquires the calculated oxygen concentration as the composition ratio of the mixed gas 31.

In step S102, the processor 17 determines whether or not the composition ratio of the mixed gas 31 is outside the explosive range. As described above, in the present example, the target value outside the explosive range is set to an oxygen concentration of 5% or more and 9% or less and a water vapor concentration of 7% or more. In addition, in the present example, the target temperature of the culture solution 18 is set to 40°C or higher. Therefore, since a saturated water vapor amount at 40°C is approximately 7% in terms of the water vapor concentration, in a case where the target temperature of the culture solution 18 is set to 40°C or higher, the water vapor concentration of the mixed gas 31 can be adjusted to 7% or more. The processor 17 compares the oxygen concentration derived from the measured values of the composition ratio measurement unit 16 with the target value. In a case where the oxygen concentration is within a range of the target value, it is determined that the composition ratio is outside the explosive range (Y in step S102), and the process proceeds to step S104.

On the other hand, in a case where the oxygen concentration exceeds 9% which is an upper limit value of the target value, the processor 17 determines that the composition ratio of the mixed gas 31 is in the explosive range (N in step S102), and the process proceeds to step S103. In step S103, the processor 17 drives the pump 22 of the supply path 14A to supply the adjustment gas 33 to the space 11A. As a result, the oxygen concentration of the mixed gas 31 decreases, and the composition ratio of the mixed gas 31 can be returned to the outside of the explosive range.

The processor 17 repeats such a composition ratio control process until the culture process is completed (Y in step S104). As a result, the composition ratio of the mixed gas 31 in the space 11A can be maintained at the target value outside the explosive range.

In addition, during the culture, the processor 17 executes the dissolution rate control process shown in FIG. 14. First, in step S201, the processor 17 measures the hydrogen dissolution rate ($Rd(H_2)$) in the culture tank 11 according to Expression (1). Then, in step S202, the processor 17 compares the measured hydrogen dissolution rate ($Rd(H_2)$) with the target value ($TRd(H_2)$), and determines whether or not the hydrogen dissolution rate ($Rd(H_2)$) is equal to or higher than the target value ($TRd(H_2)$). In a case where the hydrogen dissolution rate ($Rd(H_2)$) is lower than the target value ($TRd(H_2)$) (N in step S202), the processor 17 proceeds to step S203 and reduces the hydrogen supply amount ($Qin(H_2)$). The processor 17 maintains the hydrogen dissolution rate ($Rd(H_2)$) at or above the target value ($TRd(H_2)$) in step S203. The processor 17 repeats such process until the culture process is completed (step S204).

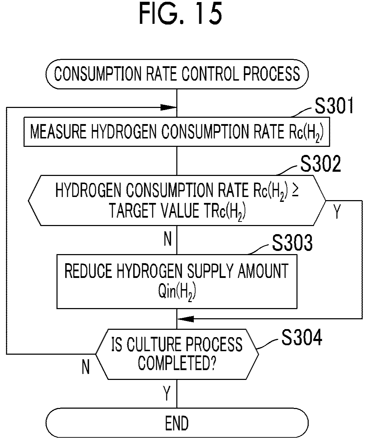

Further, the processor 17 executes the consumption rate control process shown in FIG. 15 through the dissolution rate control process. First, in step S301, the processor 17 measures the hydrogen consumption rate ($Rc(H_2)$) in the culture tank 11 according to Expression (2). Then, in step S302, the processor 17 compares the measured hydrogen consumption rate ($Rc(H_2)$) with the target value ($TRc(H_2)$), and determines whether or not the hydrogen consumption rate ($Rc(H_2)$) is equal to or higher than the target value ($TRc(H_2)$). In a case where the hydrogen consumption rate ($Rc(H_2)$) is lower than the target value ($TRc(H_2)$) (N in step S302), the processor 17 proceeds to step S303. Then, in step S303, the processor 17 reduces the hydrogen supply amount ($Qin(H_2)$) in the same manner as in step S203 of the dissolution rate control process. The processor 17 maintains the hydrogen consumption rate ($Rc(H_2)$) at or above the target value ($TRc(H_2)$) in step S303. The processor 17 repeats such process until the culture process is completed (step S304).

As described above, the culture device 10 according to the first embodiment of the disclosed technology comprises the composition ratio control mechanism of the mixed gas 31 and the dissolution rate control mechanism of the hydrogen (an example of a combustible gas). Since the composition ratio of the mixed gas 31 in the culture tank 11 is maintained at the target value outside the explosive range by the composition ratio control mechanism, the safety is improved as compared with the related art.

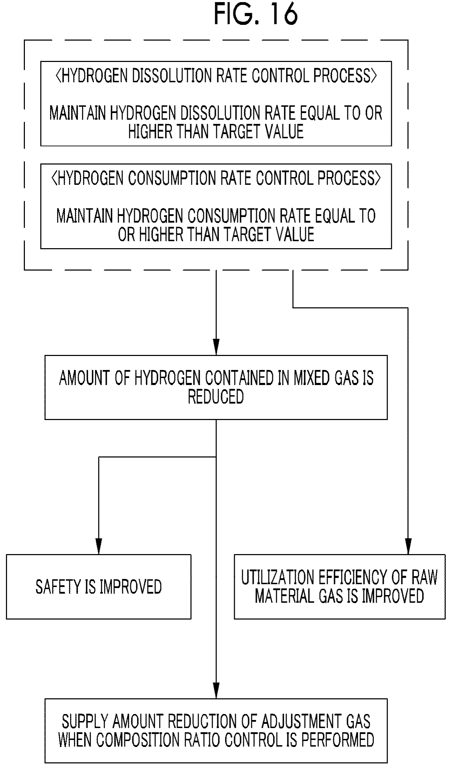

In addition, as shown in FIG. 16, since the hydrogen dissolution rate in the culture solution 18 in the culture tank 11 is maintained at or above the target value by the dissolution rate control, the amount of the hydrogen released into the space 11A is reduced, and the amount of the hydrogen contained in the mixed gas 31 is reduced. As a result, the safety is further improved. The culture device 10 according to the first embodiment performs the composition ratio control and the dissolution rate control of the mixed gas 31, so that the safety is also high as compared with a case where the dissolved oxygen concentration in the culture solution 18 is controlled alone as in JP2564008B. As shown in FIG. 16, the use efficiency of the raw material gas 26 can be improved by performing the dissolution rate control.

In addition, in the culture device 10 according to the first embodiment, the advantages of combining the composition ratio control and the dissolution rate control of the mixed gas 31 are as follows. For example, in a case where the composition ratio control of the mixed gas 31 is executed alone without performing the dissolution rate control, the supply amount of the adjustment gas 33 required to maintain the hydrogen concentration and the oxygen concentration at the target value outside the explosive range may be large. In a case where the supply amount of the adjustment gas 33 is increased, the discharge amount of the mixed gas 31 is also increased. Even in a case where the mixed gas 31 is reused, there is a limit to the amount that can be reused, and the mixed gas 31 exceeding the limit has to be released to the atmosphere. In this case, the adjustment gas 33 for inactivating the mixed gas 31 is wasted.

In a case where the composition ratio control and the dissolution rate control are combined, the amount of the hydrogen released into the space 11A is reduced. Therefore, as shown in FIG. 16, in the composition ratio control of the mixed gas 31, the supply amount of the adjustment gas 33 required to maintain the hydrogen concentration or the oxygen concentration at the target value outside the explosive range can be reduced. By combining the composition ratio control and the dissolution rate control of the mixed gas 31 in this manner, the waste of the adjustment gas 33 can be reduced.

In addition, the culture device 10 according to the first embodiment controls the supply amount of the hydrogen using an indicator of the dissolution rate instead of simply reducing the supply amount of the hydrogen. The hydrogen that is not dissolved in the culture solution 18 is not consumed by the hydrogen-oxidizing bacteria 21 to be cultured, and is wasted and released. The culture device 10 according to the first embodiment can reduce the hydrogen to be wasted and released by reducing the supply amount of the hydrogen using the dissolution rate as the indicator. Therefore, there is also little concern that the raw material of the hydrogen-oxidizing bacteria 21 will be insufficient, and the use efficiency of the hydrogen as the raw material gas 26 is also improved.

By setting the target value of the dissolution rate to 90%, the safety is further improved. Further, the use efficiency of the raw material gas 26 can be further improved by performing the hydrogen consumption rate control.

In the above example, the supply amount of the hydrogen is reduced as the dissolution rate control, but as a method of improving the dissolution rate, there is also a method of lowering the temperature of the culture solution 18. The solubility of the hydrogen can be changed by adjusting the temperature of the culture solution 18. In addition, a plurality of types of bubble generation devices (for example, the sparger 28) that generate different bubble diameters may be provided, and the dissolution rate may be controlled by selectively switching between these devices. As described above, this is because the dissolution rate is improved as the bubble diameter is smaller.

In addition, it is effective to combine the setting of the bubble diameter shown in FIG. 7 in order to maintain the dissolution rate at or above the target value. In addition, as shown in FIG. 8, it is also effective to reduce the surface tension by adding the surface tension reducer to the culture solution 18.

In addition, in the first embodiment, as an example, the hydrogen-oxidizing bacteria 21 is used as the microorganism to be cultured, and the raw material gas 26 contains hydrogen as a combustible gas and contains oxygen and carbon dioxide as other components. In a case of such a combination of the culture target and the raw material gas 26, the disclosed technology is particularly effective. This is because, first, the raw material gas 26 contains, as components, hydrogen that is a combustible gas, and oxygen that is a combustion-supporting gas. Therefore, the safety measure of suppressing the explosion is very important, so that the disclosed technology capable of further improving the safety is particularly effective.

In addition, since the raw material gas 26 contains carbon dioxide, which is an inert gas, as a component, the adjustment gas 33 can be reused as the raw material gas 26 by using the carbon dioxide as the adjustment gas 33. Therefore, the disclosed technology is also effective in such a combination in terms of the use efficiency.

In addition, in the culture device 10 according to the first embodiment, the component separation mechanism 52 shown in FIGS. 2 and 3 and the gas separation mechanism 61 shown in FIGS. 2 and 4 in the example of the common configuration are combined, which is effective in improving the safety and the use efficiency of the raw material gas 26. First, regarding the safety, since the combustible gas can be separated, the safety of the gas circulation path 39 and the gas reuse path 62 is easily ensured. In addition, by separating a specific component from the mixed gas 31 or the like, it is easy to reuse the mixed gas 31 for a specific purpose.

In addition, in the culture device 10 according to the first embodiment, the technology of disposing the mixed gas extraction port 34 of the gas circulation path 39 at a position where the foam layer 18B can be taken in as shown in FIG. 2 in the example of the common configuration is combined. Such a technology is effective in that the adverse effect on the sensor or the like is reduced and accurate control is possible.

In addition, in the culture device 10 according to the first embodiment, the concentration reduction mechanism of the culture solution 18 taken out from the culture tank 11 shown in FIG. 4 in the example of the common configuration is combined. Such a technology is effective in improving the safety in the recovery path 56 and the recovery tank 57 on the downstream side of the culture solution recovery mechanism 55.

In addition, in the culture device 10 according to the first embodiment, the explosion suppression mechanism shown in FIGS. 2 and 4 and the like in the example of the common configuration is combined. Such a technology is effective in improving the safety in the gas reuse path 62.

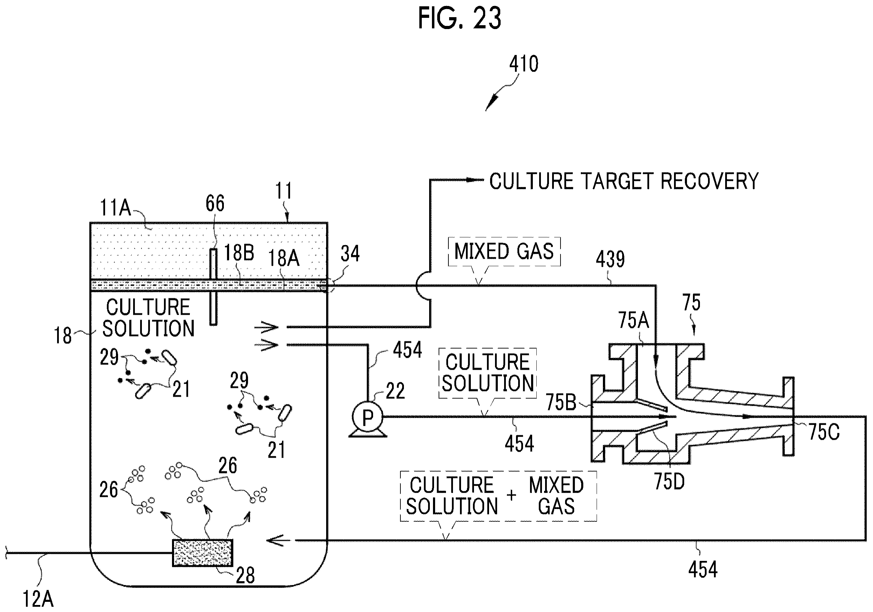

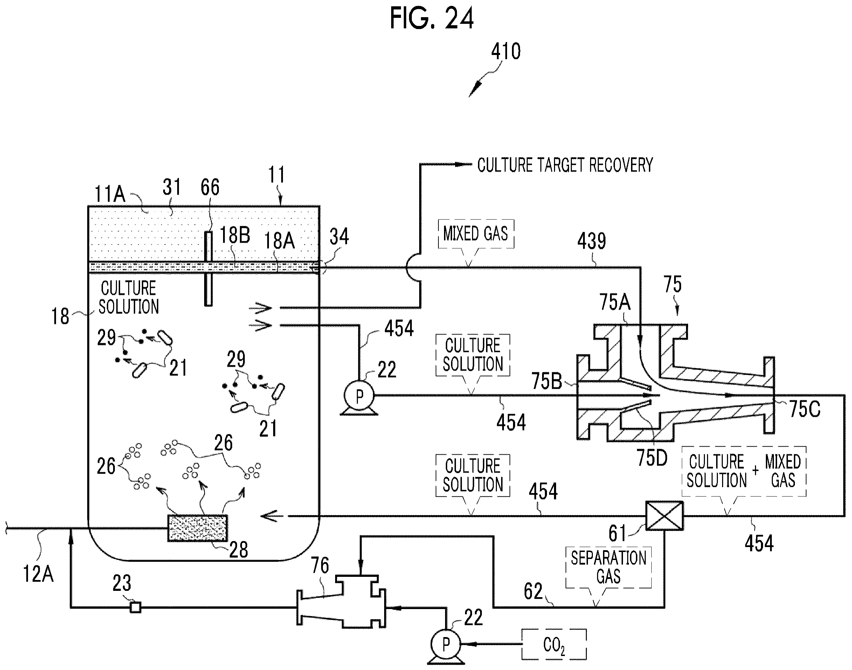

In addition, as the explosion suppression mechanism, the gas separation mechanism 61 that separates the hydrogen from the separation gas separated from the culture solution 18 has been described as an example, but other configurations can be considered as the explosion suppression mechanism. For example, a configuration in which the composition ratio of the gas to be reused is controlled to the target value outside the explosive range by adding an inert gas such as the adjustment gas 33 in a case of transferring the gas to be reused in the gas reuse path 62 is considered. In addition, a configuration in which the ignition source is excluded as much as possible from the gas reuse path 62 is also considered as the explosion suppression mechanism. For example, a pump 22 having a mechanical movable part that generates frictional heat may be an ignition source. As will be described in the fourth embodiment, the explosion suppression mechanism may be realized by a method of replacing the pump 22 with a pressure applying device (a fluid ejector 75, 76 shown in FIGS. 23 and 24) having no movable part.

It should be noted that, in the culture device 10 according to the first embodiment, not all of the elements shown as the example of the common configuration may be provided, and some of the elements may be combined.

3. Second Embodiment

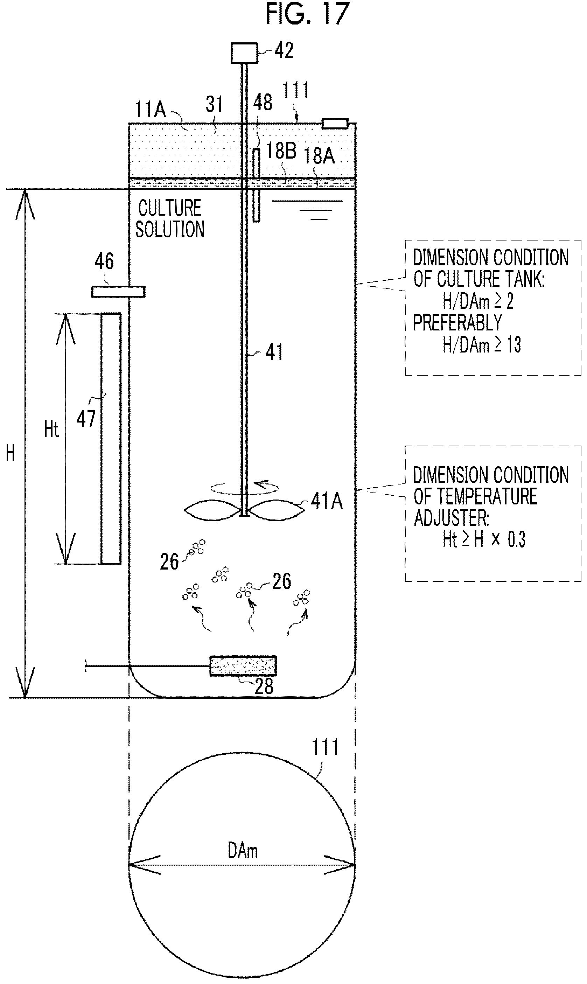

The culture device 10 according to the second embodiment will be described with reference to FIGS. 17 to 21. In the second embodiment, the description of the example of the common configuration shown in FIGS. 1 to 9 and the configuration described in the first embodiment shown in FIGS. 10 to 16 will be omitted, and the same reference numerals will be assigned.

The culture device 10 according to the second embodiment has a configuration in which the composition ratio control mechanism of the first embodiment and the vertically long culture tank 111 as shown in FIG. 17 are combined. More specifically, in a case where a circular equivalent diameter of a cross section orthogonal to the height direction of the culture tank 111 is defined as DAm and a maximum height of the liquid surface 18A is defined as H, the dimension of the culture tank 111 according to the second embodiment satisfies a dimension condition of $H/DAm \geq 2$. The height direction is a vertical direction.

Here, the "circular equivalent diameter" is a diameter of a circle in a case where the cross section is a circle, a diameter of a circumscribed circle in a case where the cross section is a polygon, and a major axis in a case of an ellipse or a long circle. The "maximum height of the liquid surface" refers to a height of the liquid surface 18A of the culture solution 18 in a case where the culture solution 18 having the maximum capacity that can be accommodated is accommodated in the culture tank 111. The maximum capacity that can be accommodated is a maximum capacity at which the culture process can be executed. The maximum height of the liquid surface 18A is a height measured in a state where various sensors such as the water level gauge 66 are immersed in the culture solution 18 and all elements that cause the liquid surface 18A to fluctuate, such as the stirring rod 41 and the sparger 28, are stopped.

By using the vertically long culture tank 111 having such a dimension condition, the dissolution rate of the combustible gas is improved. The raw material gas 26 supplied to the culture solution 18 rises toward the liquid surface 18A, and a part of the raw material gas 26 is dissolved in the culture solution 18 during the rise. The raw material gas 26 that is not dissolved is released into the space 11A above the liquid surface 18A to form a component of the mixed gas 31. Therefore, as the culture tank 111 is more vertically long, the dissolution time for the raw material gas 26 to be dissolved can be secured to be longer, so that the amount of the raw material gas 26 dissolved in the culture solution 18 is increased, and the amount of the raw material gas 26 contained in the mixed gas 31 is reduced by the amount. In a case where the raw material gas 26 contains the combustible gas, the amount of the combustible gas contained in the mixed gas 31 is reduced. As a result, the risk of the explosion is reduced, and the safety is improved.

In the second embodiment, the dissolution rate of the combustible gas is also improved by using the vertically long culture tank 111. As shown in FIG. 16, the utilization efficiency of the raw material gas 26 is also improved by improving the dissolution rate of the combustible gas. In addition, there is also an effect of reducing the supply amount of the adjustment gas 33 by combining the composition ratio control. Such an effect is the same as that of the first embodiment.

The culture tank 111 has a bottomed cylindrical shape. The cylindrical shape is more likely to reduce the unevenness of the concentration distribution of the culture solution 18 as compared with the tubular shape having a polygonal cross section. In addition, the cylindrical shape is also easily manufactured as compared with the tubular shape having a polygonal cross section.

In addition, the dimension condition is more preferably $H/DAm \geq 13$. As a result, the culture tank 111 is more vertically long, and the dissolution rate of the combustible gas is further improved.

In addition, in the second embodiment, as shown in FIG. 7 in the example of the common configuration, it is preferable that the d90 of the bubble diameter of the raw material gas 26 is 1000 $\mu$m or less. This is because, in a case where the dimension condition of the culture tank 111 is assumed to be $H/DAm = 2$, the bubbles have a size that is substantially eliminated in the vicinity of the liquid surface 18A by setting the d90 to 1000 $\mu$m or less.

In addition, in the second embodiment, it is also effective to reduce the bubble diameter by adding the surface tension reducer.

In addition, as shown in FIG. 17, it is preferable that a dimension of the temperature adjuster 47 in the height direction of the culture tank 111 is a length of 30% or more of the maximum height of the liquid surface 18A in total. That is, in a case where a length of the temperature adjuster 47 in the height direction is defined as Ht, it is preferable to satisfy a condition of $Ht/H \geq 30\%$. Here, the meaning of "as a whole" includes a case where a continuous length Ht of one temperature adjuster 47 is 30% or more and a case where a plurality of temperature adjusters 47 are disposed in a divided manner, in which the total length of each divided portion is 30% or more.

In a case where the culture tank 111 is vertically long, it is preferable that the temperature adjuster 47 also has a length of 30% or more of the height H of the liquid surface 18A from the viewpoint of reducing the temperature unevenness in the height direction.

In addition, the temperature adjuster 47 may have various forms, but may be disposed to surround the outer periphery of the culture tank 111. For example, in a case where the culture tank 111 has a cylindrical shape, the temperature adjuster 47 also has a cylindrical shape. Alternatively, a plurality of temperature adjusters 47 having a circular arc-shaped cross section may be disposed on the outer periphery of the culture tank 111.

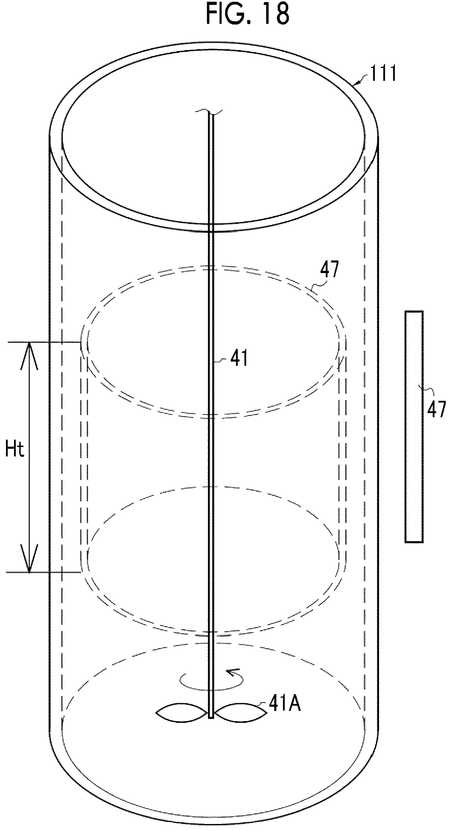

In addition, as in the modification example shown in FIG. 18, the temperature adjuster 47 may be disposed in the culture tank 111. Of course, as shown in FIG. 18, the temperature adjuster 47 may be disposed on both the inside and the outside of the culture tank 111.

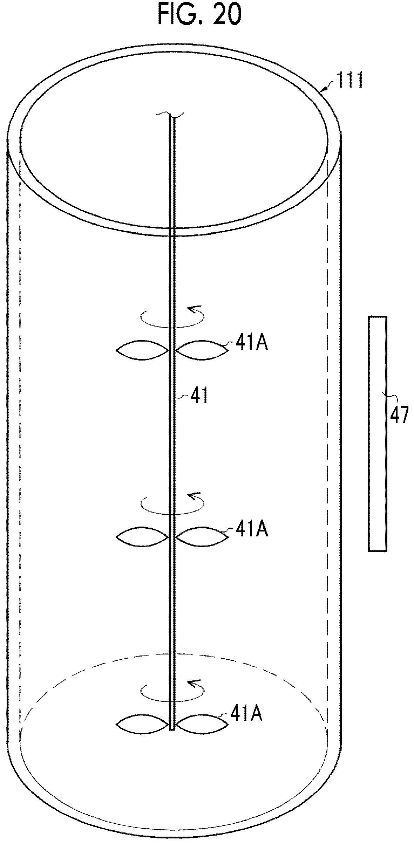

As shown in FIG. 18, it is preferable that at least a part of the temperature adjuster 47 is disposed in the culture tank 111. This is because the temperature adjuster 47 adjusts the temperature of the culture solution 18 in the culture tank 111, and thus the energy efficiency is better in a case where the temperature adjuster 47 is disposed in the culture tank 111 as compared with a case where the temperature adjuster 47 is disposed outside the culture tank 111. A form of the temperature adjuster 47 in the culture tank 111 is, for example, a cylindrical shape. Of course, a plurality of temperature adjusters 47 having a circular arc shape may be disposed. It should be noted that, in FIGS. 18 and 20 described below, the shape of the temperature adjuster 47 disposed on the outside of the culture tank 111 is shown in a simplified manner. In practice, the temperature adjuster 47 disposed on the outside of the culture tank 111 has, for example, a circular arc-shaped cross section in a horizontal direction orthogonal to the height direction and is curved along a curved surface of the outer peripheral surface of the culture tank 111.

As described above, the temperature adjuster 47 is controlled by the processor 17, and the processor 17 adjusts the temperature of the culture solution 18 to a temperature suitable for the culture through the temperature adjuster 47. The temperature of the culture solution 18 may be kept at a constant temperature during the culture period, or the temperature may be changed according to the progress of the culture.

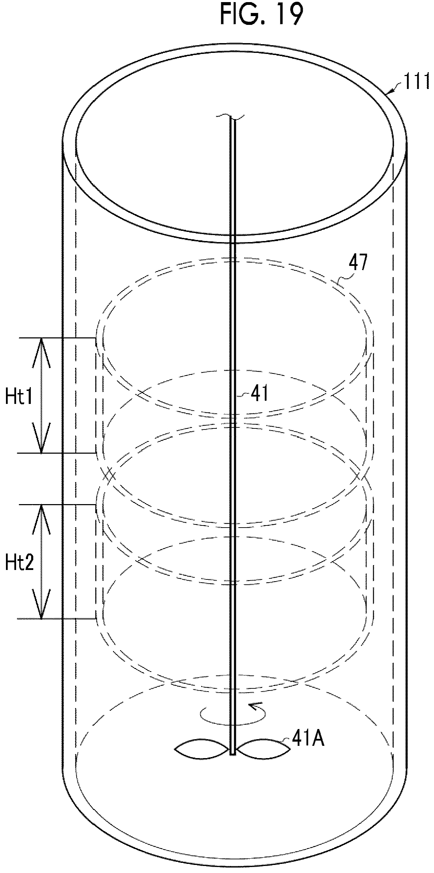

In addition, as in the modification example shown in FIG. 19, a plurality of temperature adjusters 47 may be provided, and the plurality of temperature adjusters 47 may be disposed at different positions in the height direction of the culture tank 111. Since the culture tank 11 is vertically long, it is preferable from the viewpoint of reducing the temperature unevenness in the height direction that the plurality of temperature adjusters 47 are disposed at different heights. It should be noted that, in FIG. 19, reference numerals Ht1 and Ht2 indicate lengths of two temperature adjusters 47 arranged in the height direction.

In addition, as in the modification example shown in FIG. 20, the stirring rod 41 includes a plurality of screws 41A, and the plurality of screws 41A may be disposed at different positions in the height direction of the culture tank 111. The screw 41A is an example of a "stirring unit" according to the disclosed technology, and the stirring rod 41 is an example of a "stirring mechanism".

It is preferable that the plurality of screws 41A are disposed at different heights because the concentration unevenness of the substance dissolved or dispersed in the culture solution 18 in the height direction is reduced. The substance dissolved or dispersed in the culture solution 18 is the culture target (microorganism or cell) such as the hydrogen-oxidizing bacteria 21 and the raw material gas 26.

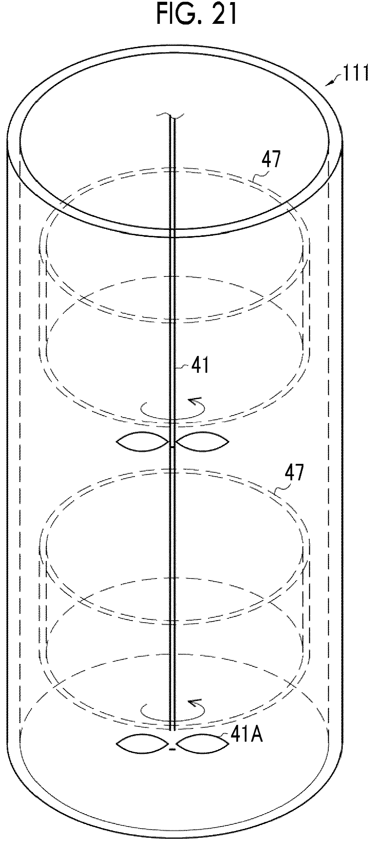

In addition, as shown in FIG. 21, in a case where a plurality of temperature adjusters 47 and the plurality of screws 41A are provided, a plurality of pairs, each including at least one temperature adjuster 47 and at least one screw 41A may be disposed at different positions in the height direction of the culture tank 111. In this manner, both the temperature unevenness and the concentration unevenness can be reduced.

It should be noted that the dissolution rate control mechanism of the first embodiment may be combined with the culture device 10 according to the second embodiment. By combining the vertically long culture tank 111 and the dissolution rate control mechanism, the amount of the combustible gas contained in the mixed gas 31 can be further reduced. As a result, the risk of the explosion of the mixed gas 31 is reduced, and the safety is further improved. In addition, since the consumption rate of the raw material gas 26 such as the hydrogen consumption rate of the hydrogen-oxidizing bacteria 21 is also improved by further improving the dissolution rate of the combustible gas, the use efficiency of the raw material gas 26 is also improved.

In a case of performing the dissolution rate control, the target value of the dissolution rate is preferably 90% or more as in the first embodiment. The effect of improving the safety and the use efficiency of the raw material gas 26 is more reliable.

In addition, as in the first embodiment, the consumption rate control of the combustible gas may be combined with the dissolution rate control. As a result, the effect of improving the use efficiency of the raw material gas 26 is more reliable.

In addition, in the second embodiment, as an example, the hydrogen-oxidizing bacteria 21 is used as the microorganism to be cultured, and the raw material gas 26 contains hydrogen as a combustible gas and contains oxygen and carbon dioxide as other components. In a case of such a combination of the culture target and the raw material gas 26, the disclosed technology is particularly effective. The reason is as described in the first embodiment.

It should be noted that, in the culture device 10 according to the second embodiment, not all of the elements shown as the example of the common configuration may be provided, and some of the elements may be combined. In addition, as in the example of the combination with the dissolution rate control, each configuration of the second embodiment and the first embodiment may be appropriately combined.

4. Third Embodiment

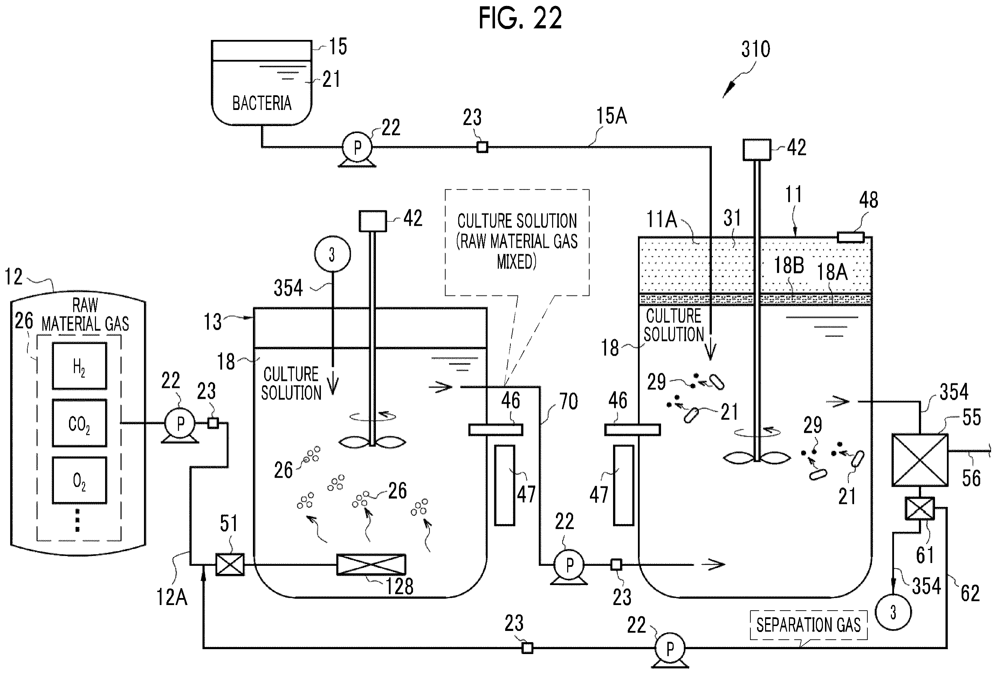

The culture device 310 according to the third embodiment will be described with reference to FIG. 22. In the third embodiment, the above-described configurations such as the example of the common configuration shown in FIGS. 1 to 9 and the first embodiment shown in FIGS. 10 to 16 will be omitted, and the same reference numerals will be assigned.

As shown in FIG. 22, the culture device 310 according to the third embodiment includes a bubble generation device 128 that mixes the raw material gas 26 with the culture solution 18 before being supplied to the culture tank 11, and a culture solution supply path 70 that is disposed between the culture solution tank 13 and the culture tank 11 and that supplies the culture solution 18 mixed with the raw material gas 26 by the bubble generation device 128 to the culture tank 11, the culture solution supply path 70 being provided separately from the supply path 15A that supplies the hydrogen-oxidizing bacteria 21 to the culture tank 11.

In the culture device 310, for example, the bubble generation device 128 is provided in the culture solution tank 13. The supply path 12A of the raw material gas 26 is connected to the bubble generation device 128 in the culture solution tank 13. The culture solution 18 mixed with the raw material gas 26 in the culture solution tank 13 is supplied to the culture tank 11 through the culture solution supply path 70. A bubble generation device such as the sparger 28 is not provided in the culture tank 11. The hydrogen-oxidizing bacteria 21 is supplied to the culture tank 11 through the supply path 15A as the culture target supply path.

In a case where the bubbles of the raw material gas 26 generated by the bubble generation device 128 and the culture target (microorganism or cell) such as the hydrogen-oxidizing bacteria 21 come into contact with each other, the culture target is damaged. In a case where the culture target is damaged by the bubbles, the culture target may die, and the culture target in the culture solution 18 may be reduced. In a case where the culture target in the culture solution 18 is reduced, the consumption rate of the combustible gas contained in the raw material gas 26, such as the hydrogen consumption rate, is also reduced. In a case where the consumption rate of the combustible gas is reduced, the amount of the combustible gas contained in the mixed gas 31 is increased. As a result, the risk of the explosion is increased, and the safety is reduced.

In the culture device 310 according to the third embodiment, the culture solution supply path 70 that supplies the culture solution 18 mixed with the raw material gas 26 in advance is provided separately from the supply path 15A of the hydrogen-oxidizing bacteria 21, so that the opportunity for the bubbles of the raw material gas 26 and the hydrogen-oxidizing bacteria 21 to come into contact with each other in the culture tank 11 is reduced. As a result, the damage to the hydrogen-oxidizing bacteria 21 from the bubbles is reduced, and the reduction of the hydrogen-oxidizing bacteria 21 in the culture solution 18 is suppressed. In a case where the reduction of the hydrogen-oxidizing bacteria 21 is suppressed, the consumption rate of the combustible gas such as the hydrogen consumption rate is improved, and the amount of the combustible gas contained in the mixed gas 31 is reduced. As a result, the risk of the explosion is reduced, and the safety is improved.

Further, in a case where the damage to the hydrogen-oxidizing bacteria 21 is reduced, the hydrogen consumption rate is increased, and the use efficiency and the culture efficiency of the raw material gas 26 are also improved.

In addition, as shown in FIG. 22, in the culture device 310 according to the third embodiment, the temperature adjuster 47 is provided in the culture solution tank 13 in addition to the culture tank 11. As a result, the temperature of the culture solution 18 in the culture tank 11 and the temperature in the culture solution tank 13 are adjusted to be the same temperature.

In the culture device 310, the culture solution 18 to which the hydrogen-oxidizing bacteria 21 is supplied is in the culture tank 11, and the culture solution 18 to which the raw material gas 26 is supplied is in the culture solution tank 13. The culture solution 18 in each of the culture solution tank 13 and the culture tank 11 is mixed through the culture solution supply path 70, but in a case where there is a temperature difference between the two, the concentration unevenness of the raw material gas 26 may occur in the culture solution 18 after mixing due to the temperature difference. In a case where there is a concentration unevenness of the raw material gas 26, the hydrogen-oxidizing bacteria 21 in a region having a low concentration of the raw material gas 26 may also die. By providing the temperature adjuster 47 in each of the culture tank 11 and the culture solution tank 13, the damage to the culture target such as the hydrogen-oxidizing bacteria 21 due to the temperature difference of the culture solution 18 can be reduced. As a result, the hydrogen consumption rate by the hydrogen-oxidizing bacteria 21 is improved, and the amount of hydrogen contained in the mixed gas 31 is also reduced, so that the safety is further improved. Similarly, the use efficiency and the culture efficiency of the raw material gas 26 are also improved.

In addition, since the mixing of the raw material gas 26 and the culture solution 18 is performed before being supplied to the culture tank 11, for example, the bubble generation device 128 can be provided in the culture solution supply path 70. However, as shown in FIG. 22, it is easier to install the bubble generation device 128 in the culture solution tank 13 than in the culture solution supply path 70 or the like because there is more space.

The bubble generation device 128 may be the sparger 28, but may be a vortex shear type that generates bubbles by vortex shear force, or the above-described ejector type or static mixer type. The bubble generation device of these types has a larger output than the sparger 28 and can supply a larger amount of the raw material gas 26, so that the mass productivity is excellent. Therefore, the culture efficiency can be improved. On the other hand, as the output of the bubble generation device 128 is higher, the damage to the hydrogen-oxidizing bacteria 21 due to the bubbles is increased. In the third embodiment, the raw material gas 26 and the culture solution 18 can be mixed without considering the contact between the bubbles of the raw material gas 26 and the hydrogen-oxidizing bacteria 21. Therefore, a high-output bubble generation device 128 such as the vortex shear type can be used.

In addition, the culture device 310 according to the third embodiment also includes a culture solution circulation path 354 that takes out a part of the culture solution 18 from the culture tank 11, recovers a part of the culture target such as the hydrogen-oxidizing bacteria 21 from the taken-out culture solution 18, and returns the remaining culture solution 18 after the recovery to the culture tank 11, as in the first embodiment. The culture solution circulation path 354 according to the third embodiment includes the culture solution supply path 70 that connects the culture solution tank 13 and the culture tank 11. The culture solution circulation path 354 according to the third embodiment is different from the culture solution circulation path 54 according to the first embodiment in that the culture solution is returned to the culture tank 11 through the culture solution tank 13. The culture solution 18 can be perfused while culturing by the culture solution circulation path 354 to perform perfusion culture. Even in a case of performing the perfusion culture, the culture solution 18 after the hydrogen-oxidizing bacteria 21 is recovered is returned to the culture solution tank 13, so that the contact between the bubbles of the raw material gas 26 and the hydrogen-oxidizing bacteria 21 is reduced. As a result, the damage to the hydrogen-oxidizing bacteria 21 from the bubbles is small.

In addition, in the third embodiment, as an example, the hydrogen-oxidizing bacteria 21 is used as the microorganism to be cultured, and the raw material gas 26 contains hydrogen as a combustible gas and contains oxygen and carbon dioxide as other components. In a case of such a combination of the culture target and the raw material gas 26, the disclosed technology that aims to improve the safety as in the first embodiment is particularly effective.

In addition, the hydrogen-oxidizing bacteria 21 consume hydrogen and carbon dioxide as an energy source and a carbon source, respectively. As described above, in the culture device 310 according to the third embodiment, since the raw material gas 26 and the culture solution 18 are mixed in the culture solution tank 13, it is not necessary to consider the contact between the hydrogen-oxidizing bacteria 21 and the bubbles of the raw material gas 26, and the high-output bubble generation device 128 can be used. Therefore, the raw material gas 26 can be sufficiently supplied to and dissolved in the culture solution 18 as compared with the sparger 28 (see FIG. 2). Therefore, the culture device 310 according to the third embodiment can provide sufficient nutrition to the hydrogen-oxidizing bacteria 21, which is advantageous in terms of the culture efficiency.

In addition, in the third embodiment, as shown in FIG. 3 as an example of the common configuration, the raw material gas 26 may be supplied such that each component of hydrogen, oxygen, and carbon dioxide is separately supplied. As a result, the concentration distribution of each component is unlikely to occur in the culture solution 18, and the raw material gas 26 is unlikely to be insufficient for the hydrogen-oxidizing bacteria 21, and the damage to the hydrogen-oxidizing bacteria 21 is small.

In addition, in the third embodiment, as shown in FIG. 7 as an example of the common configuration, the d90 of the bubble diameter of the raw material gas 26 may be 1000 $\mu$m or less. As described above, the dissolution rate can be further improved by reducing the bubble diameter. As a result, the safety and the use efficiency of the raw material gas 26 are improved.

In addition, in the third embodiment, the composition ratio control shown in the first embodiment may be performed. In this manner, the safety is further improved.

It should be noted that, in the culture device 310 according to the third embodiment, not all of the elements shown as the example of the common configuration may be provided, and some of the elements may be combined. In addition, each configuration of the third embodiment and the first embodiment and the second embodiment may be appropriately combined, such as the combination of the dissolution rate control and the vertically long culture tank 111.


5. Fourth Embodiment


The culture device 410 according to the fourth embodiment will be described with reference to FIGS. 23 and 24. In the fourth embodiment, the above-described configurations such as the example of the common configuration shown in FIGS. 1 to 9 and the first embodiment shown in FIGS. 10 to 16 will be omitted, and the same reference numerals will be assigned.

As shown in FIG. 23, the culture device 410 according to the fourth embodiment includes a gas circulation path 439 for reusing the mixed gas 31 in the space 11A as the raw material gas 26, and a fluid ejector 75 that is disposed on the gas circulation path 439 and that applies a circulation pressure to the mixed gas 31 to circulate the mixed gas 31. The fluid ejector 75 is an example of a pressure applying device that applies a circulation pressure to the mixed gas 31 by a drive fluid input from the outside without having a mechanical movable part. The pump 22 is a pressure applying device, but has a mechanical movable part such as a rotating body, and the mixed gas 31, which is a target to which the circulation pressure is applied, and the mechanical movable part come into contact with each other. The pump 22 is distinguished from the pressure applying device having no mechanical movable part in the present application in that the pump 22 has a mechanical movable part.

The fluid ejector 75 includes a first input port 75A into which the mixed gas 31 is input, a second input port 75B into which the drive fluid is input, and an output port 75C from which the mixed gas 31 is output. A nozzle 75D that jets the drive fluid is provided at the back of the second input port 75B.

The nozzle 75D has a tapered shape toward a distal end where a jetting port is formed. The distal end of the nozzle 75D is disposed at a location where a flow passage from the first input port 75A and a flow passage from the second input port 75B are combined. In a case where the drive fluid is input to the second input port 75B from the outside, the drive fluid passes through the nozzle 75D, and the flow rate increases due to the tapered shape of the nozzle 75D, and the drive fluid is jetted in this state. Since the flow rate of the drive fluid is high and the pressure is low, the mixed gas 31 input from the first input port 75A is sucked into the drive fluid and is output from the output port 75C together with the drive fluid. As a result, the circulation pressure is applied to the mixed gas 31.

The drive fluid is, for example, the culture solution 18 taken out from the culture tank 11. The culture solution 18 as the drive fluid is pressurized by the pump 22 and is input to the fluid ejector 75. The culture solution 18 and the mixed gas 31 are output from the output port 75C of the fluid ejector 75 in a mixed state. A culture solution circulation path 454 is connected to the output port 75C. The culture solution 18 and the mixed gas 31 are returned to the culture tank 11 through the culture solution circulation path 454. As a result, the mixed gas 31 is circulated. In the present example, the culture solution circulation path 454 is also used as the gas circulation path 439.

As described above, in the culture device 410 according to the fourth embodiment, the mixed gas 31 is circulated by providing the fluid ejector 75 in the gas circulation path 439 through which the mixed gas 31 is circulated. The fluid ejector 75 is an example of a pressure applying device that does not have a mechanical movable part, and since the fluid ejector 75 does not have a mechanical movable part, heat generation due to friction does not occur, and the fluid ejector 75 does not become an ignition source such as high temperature, sparks, and static electricity. The ignition source is one of the three elements of the explosion. Therefore, in the culture device 410, even in a case of reusing the mixed gas 31 as the raw material gas 26, the risk of the explosion is low, and the safety is improved as compared with the related art.

In addition, in the culture device 410, the pump 22 having the mechanical movable part is provided in order to apply the pressure to the culture solution 18 as the drive fluid. However, since the pump 22 is not disposed in the gas circulation path 439 through which the mixed gas 31 is circulated, the mixed gas 31 and the pump 22 do not come into contact with each other, and the pump 22 does not become an ignition source that causes the explosion of the mixed gas 31.

As described above, in the culture device 410 according to the fourth embodiment, a device that may be an ignition source, such as the pump 22, is excluded from the gas circulation path 439 through which the mixed gas 31 is circulated. Therefore, even in a case where the composition ratio of the mixed gas 31 is in the explosive range, the safety is high as compared with a case where the pump 22 is disposed in the gas circulation path 439.

In the culture device 410, the culture solution 18 is used as the drive fluid. The culture solution 18 is also a substance that can be reused in the culture process by being circulated in the same manner as the mixed gas 31. Therefore, in a case where the culture solution 18 is used as the drive fluid, there is less waste as compared with a case where a dedicated fluid that cannot be used for the culture process is used as the drive fluid. In addition, since the mixed gas 31 is dissolved in the culture solution 18 and is reused, it is also advantageous from the viewpoint of preventing the impurities from being mixed into the raw material gas 26 to use the culture solution 18 as the drive fluid.

In addition, as described above, in the culture device 410 according to the fourth embodiment, the safety is relatively high even in a case where the composition ratio of the mixed gas 31 is in the explosive range. Therefore, the supply amount of the raw material gas 26 can also be decided based on the standard that emphasizes the culture efficiency of the hydrogen-oxidizing bacteria 21 rather than the safety.

For example, in the culture device 410, during the culture, the consumption amount of the raw material gas 26 consumed by the hydrogen-oxidizing bacteria 21 in the raw material gas 26 supplied to the culture tank 11 may be measured, and the raw material gas 26 in an amount equal to or greater than the consumption amount of the raw material gas 26 may be supplied. In the culture device 410, since the safety is high, it is easy to perform such control. The consumption amount of the raw material gas 26 can be measured by the same procedure as the procedure described in FIG. 12 in the first embodiment. The raw material gas supply unit including the processor 17 calculates the raw material gas 26 having a consumption amount equal to or greater than the measured consumption amount of the raw material gas 26, and decides the supply amount. By such control, the raw material gas 26 can be sufficiently supplied.

Specifically, the supply amount of the raw material gas 26 supplied to the culture tank 11 through the raw material gas supply unit and the gas circulation path 39 may be 2 to 15 times the consumption amount of the raw material gas 26. That is, the total supply amount of the raw material gas 26 supplied to the culture tank 11 is controlled to be 2 to 15 times the consumption amount of the raw material gas 26 in the culture tank 11. In a case where the total supply amount of the raw material gas 26 is set to this degree, a sufficient amount of the raw material gas 26 required by the hydrogen-oxidizing bacteria 21 can be provided, and the raw material gas 26 is not insufficient. In addition, in a case where such a sufficient amount of the raw material gas 26 is provided, the concentration unevenness of the raw material gas 26 in the culture tank 11 is also reduced. As a result, the culture efficiency can be improved.

In addition, of course, the culture device 410 according to the fourth embodiment can be appropriately combined with the configurations of each of the above-described embodiments. For example, the composition ratio control of maintaining the composition ratio of the mixed gas 31 shown in FIG. 11 at the target value in the explosive range may be performed. In the fourth embodiment, since the ignition source is excluded from the gas circulation path 439, the safety is high even in a case where the composition ratio of the mixed gas 31 is in the explosive range, but the safety is further improved by performing the composition ratio control of the mixed gas 31.

In addition, as shown in FIG. 23, a configuration in which the mixed gas extraction port 34 of the gas circulation path 439 is disposed at a position where the foam layer 18B can be taken in may be adopted. As a result, as shown in the example of the common configuration, the adverse effect of the foam layer 18B can be reduced.

In addition, in the fourth embodiment, as an example, the hydrogen-oxidizing bacteria 21 is used as the microorganism to be cultured, and the raw material gas 26 contains hydrogen as a combustible gas and contains oxygen and carbon dioxide as other components. In a case of such a combination of the culture target and the raw material gas 26, the disclosed technology is particularly effective. As described above, the raw material gas 26 contains, as components, hydrogen that is a combustible gas, and oxygen that is a combustion-supporting gas. Therefore, the safety measure of suppressing the explosion is very important, so that the disclosed technology capable of further improving the safety is particularly effective.

It should be noted that, in the present example, the output port 75C of the fluid ejector 75 is connected to the culture tank 11 through the pipe of the culture solution circulation path 454, but the output port 75C may be directly connected to the culture tank 11 without using the pipe.

It should be noted that, in the present example, the fluid ejector 75 is used as the pressure applying device that does not have a mechanical movable part. A large number of existing products of the fluid ejector 75 are also easily used. Of course, as the pressure applying device that does not have a mechanical movable part, for example, a vortex shear type pressure applying device can be used instead of the fluid ejector 75. The vortex shear type pressure applying device generates a high-speed swirling flow by causing the drive fluid to vortex, and applies the circulation pressure to the mixed gas 31 by the pressure of the high-speed swirling flow. In addition, as the pressure applying device, for example, a bubble generation device "YJ nozzle" manufactured by Enviro Vision Co., Ltd. can also be used. The "YJ nozzle" can be used as a bubble generation device, but can also be used as a pressure applying device that applies a pressure to a fluid by using a pressure change in a flow passage, as in the fluid ejector 75 shown in FIG. 23.

In addition, in the example of FIG. 23, the culture solution 18 and the mixed gas 31 are returned to the culture tank 11 in a mixed state, but as shown in FIG. 24, the culture solution 18 and the mixed gas 31 may be separated and returned to the culture tank 11.

In this case, the culture solution 18 and the mixed gas 31 are output from the output port 75C of the fluid ejector 75 in a mixed state, and the culture solution 18 and the mixed gas 31 are separated by the gas separation mechanism 61. Then, the separated culture solution 18 is returned to the culture tank 11 through the culture solution circulation path 454. On the other hand, the separated separation gas is returned to the culture tank 11 through the gas reuse path 62. For example, a fluid ejector 76 that applies a circulation pressure to the separation gas is disposed in the gas reuse path 62. As an example, carbon dioxide is used as the drive fluid in the fluid ejector 76. Since the carbon dioxide is a component of the separation gas, the separation gas is not mixed with impurities. The separation gas is returned to the culture tank 11 through the fluid ejector 76.

In each embodiment described above, the hydrogen-oxidizing bacteria 21 has been described as an example of microorganisms, but the technology of the present disclosure can also be applied to microorganisms other than the hydrogen-oxidizing bacteria 21 or to cells. As the microorganisms, for example, acetogenic bacteria such as Clostridium autoethanogenum, or methanotrophic bacteria such as Methylococcus capsulatus may be used. In a case of the acetogenic bacteria, the components of the raw material gas 26 are hydrogen ($H_2$), carbon monoxide (CO), and carbon dioxide ($CO_2$), which are combustible gases. In a case of the methanotrophic bacteria, the components of the raw material gas 26 are methane ($CH_4$), which is a combustible gas, and air. In addition, the cell is a morphological and/or functional unit constituting a living body, and the living body from which the cell originates is not particularly limited, but may be an animal or a plant. The cell may be an established cell or a genetically modified cell, and may include an artificial cell. Examples of the cells include Chinese hamster ovary cells (CHO cells), human embryonic kidney cells 293 (HEK293 cells), and Spodoptera frugiperda cells 9 (Sf9 cells).

(Regarding Supplementary Notes)

In addition, from the description of the first to fourth embodiments, the inventions described in the following First to Fourth Supplementary Notes can be understood.

(First Supplementary Notes)

[First Supplementary Note 1]

A culture device that cultures a microorganism or a cell that uses a raw material gas containing a combustible gas for proliferation or production of an organic substance, the culture device comprising:

a culture tank that accommodates a culture solution for culturing the microorganism or the cell;
a raw material gas supply unit that supplies the raw material gas to the culture tank;
a composition ratio control mechanism that measures a composition ratio of a mixed gas in a space above a liquid surface of the culture solution in the culture tank and supplies an adjustment gas into the space in accordance with the measured composition ratio of the mixed gas to maintain the composition ratio of the mixed gas in the space at a target value outside a preset explosive range;
a gas circulation path for reusing the mixed gas in the space as the raw material gas; and
a dissolution rate control mechanism that measures, during the culture, a combustible gas dissolution rate, which is a proportion of a combustible gas dissolved in the culture solution, to the combustible gas contained in the raw material gas supplied to the culture tank through the gas circulation path or the raw material gas supply unit, and maintains the measured combustible gas dissolution rate at or above a preset target value.

[First Supplementary Note 2]

The culture device according to the First Supplementary Note 1,
in which the target value of the combustible gas dissolution rate is 90% or more.

[First Supplementary Note 3]

The culture device according to the First Supplementary Note 1 or 2,
in which the dissolution rate control mechanism further measures, during the culture, a combustible gas consumption rate, which is a proportion of the combustible gas consumed by the microorganism or the cell to the combustible gas contained in the raw material gas supplied to the culture tank through the gas circulation path and the raw material gas supply unit, and maintains the measured combustible gas consumption rate at 90% or more through control of the combustible gas dissolution rate.

[First Supplementary Note 4]

The culture device according to any one of the First Supplementary Notes 1 to 3,
in which d90 of a bubble diameter of the raw material gas generated in the culture solution is 1000 $\mu$m or less.

[First Supplementary Note 5]

The culture device according to any one of the First Supplementary Notes 1 to 4,
in which the culture solution contains a surface tension reducer.

[First Supplementary Note 6]

The culture device according to any one of the First Supplementary Notes 1 to 5,
in which a surface tension of the culture solution is 65 mN/m or less.

[First Supplementary Note 7]

The culture device according to any one of the First Supplementary Notes 1 to 6,

in which the microorganism is a hydrogen-oxidizing bacterium,
the raw material gas contains hydrogen as the combustible gas and contains oxygen and carbon dioxide as other components, and
the adjustment gas is carbon dioxide.

[First Supplementary Note 8]

The culture device according to any one of the First Supplementary Notes 1 to 7, further comprising:
a first component separation mechanism that separates a specific component from a mixed gas taken out from the culture tank, or a second component separation mechanism that separates a specific component from a separation gas separated from the culture solution taken out from the culture tank.

[First Supplementary Note 9]

The culture device according to any one of the First Supplementary Notes 1 to 8,
in which, in the gas circulation path, a mixed gas extraction port that takes out the mixed gas from the culture tank is disposed at a position allowing a foam layer present on the liquid surface in the culture tank to be taken in.

[First Supplementary Note 10]

The culture device according to any one of the First Supplementary Notes 1 to 9, further comprising:

a culture solution recovery mechanism that recovers a recovery solution, which is a part of the culture solution containing the produced organic substance, from the culture solution taken out from the culture tank;
a recovery solution tank that stores the recovery solution containing the organic substance; and
a concentration reduction mechanism that reduces a dissolved combustible gas concentration in the recovery solution stored in the recovery solution tank to 1/10 or less of a dissolved combustible gas concentration in the culture solution in the culture tank.

[First Supplementary Note 11]

The culture device according to any one of the First Supplementary Notes 1 to 10, further comprising:
an explosion suppression mechanism that suppresses an explosion in a gas reuse path for reusing a gas separated from the culture solution.

[First Supplementary Note 12]

A method for controlling a culture device that cultures a microorganism or a cell and that includes a culture tank that accommodates a culture solution for culturing a microorganism or a cell that uses a raw material gas containing a combustible gas for proliferation or production of an organic substance, and a raw material gas supply unit that supplies the raw material gas containing a combustible gas to the culture tank, the method including:

measuring a composition ratio of a mixed gas in a space above a liquid surface of the culture solution in the culture tank and supplying an adjustment gas into the space in accordance with the measured composition ratio of the mixed gas to maintain the composition ratio of the mixed gas in the space at a target value outside a preset explosive range; and
measuring, during the culture, a combustible gas dissolution rate, which is a proportion of a combustible gas dissolved in the culture solution, to the combustible gas contained in the raw material gas supplied to the culture tank through a gas circulation path for reusing the mixed gas in the space as the raw material gas or the raw material gas supply unit, and maintaining the measured combustible gas dissolution rate at a preset target value.

(Second Supplementary Notes)

[Second Supplementary Note 1]

A culture device that cultures a microorganism or a cell that uses a raw material gas containing a combustible gas for proliferation or production of an organic substance, the culture device comprising: a culture tank that accommodates a culture solution for culturing the microorganism or the cell; a raw material gas supply unit that supplies the raw material gas to the culture tank; and a composition ratio control mechanism that measures a composition ratio of a mixed gas in a space above a liquid surface of the culture solution in the culture tank and supplies an adjustment gas into the space in accordance with the measured composition ratio of the mixed gas to maintain the composition ratio of the mixed gas in the space at a target value outside a preset explosive range, in which, in a case where a circular equivalent diameter of a cross section orthogonal to a height direction of the culture tank is defined as DAm and a maximum height of the liquid surface is defined as H, a dimension of the culture tank satisfies a condition of $H/DAm \geq 2$.

[Second Supplementary Note 2]

The culture device according to the Second Supplementary Note 1,
in which the culture tank has a bottomed cylindrical shape.

[Second Supplementary Note 3]

The culture device according to the Second Supplementary Note 1 or 2,
in which the condition is $H/DAm \geq 13$.

[Second Supplementary Note 4]

The culture device according to any one of the Second Supplementary Notes 1 to 3,
in which the d90 of a bubble diameter of the raw material gas generated in the culture solution is 1000 $\mu$m or less.

[Second Supplementary Note 5]

The culture device according to any one of the Second Supplementary Notes 1 to 4, further comprising:

a temperature adjuster that adjusts a temperature of the culture solution,
in which at least a part of the temperature adjuster is disposed in the culture tank.

[Second Supplementary Note 6]

The culture device according to the Second Supplementary Note 5,
in which a dimension of the temperature adjuster in the height direction of the culture tank is a length of 30% or more of the maximum height of the liquid surface in total.

[Second Supplementary Note 7]

The culture device according to the Second Supplementary Note 5 or 6,
in which a plurality of temperature adjusters are provided, and each of the plurality of temperature adjusters is disposed at a different position in the height direction of the culture tank.

[Second Supplementary Note 8]

The culture device according to any one of the Second Supplementary Notes 5 to 7, further comprising:

a stirring mechanism that includes a plurality of stirring units that stir the culture solution,
in which the plurality of stirring units are disposed at different positions in the height direction of the culture tank.

[Second Supplementary Note 9]

The culture device according to the Second Supplementary Note 8,
in which, in a case where a plurality of temperature adjusters and the plurality of stirring units are provided, a plurality of pairs, each including at least one temperature adjuster and at least one stirring unit, are disposed at different positions in the height direction of the culture tank.

[Second Supplementary Note 10]

The culture device according to any one of the Second Supplementary Notes 1 to 9, further comprising:

a gas circulation path for reusing the mixed gas in the space as the raw material gas; and
a dissolution rate control mechanism that measures, during the culture, a combustible gas dissolution rate, which is a proportion of the combustible gas dissolved in the culture solution, to the combustible gas contained in the raw material gas supplied to the culture tank through the gas circulation path and the raw material gas supply unit, and maintains the measured combustible gas dissolution rate at a preset target value.

[Second Supplementary Note 11]

The culture device according to the Second Supplementary Note 10,
in which the target value of the combustible gas dissolution rate is 90% or more.

[Second Supplementary Note 12]

The culture device according to the Second Supplementary Note 10 or 11,
in which the dissolution rate control mechanism further measures, during the culture, a combustible gas consumption rate of the combustible gas consumed by the microorganism or the cell to the combustible gas contained in the raw material gas supplied to the culture tank through the gas circulation path or the raw material gas supply unit, and maintains the measured combustible gas consumption rate at 90% or more through control of the combustible gas dissolution rate.

[Second Supplementary Note 13]

The culture device according to any one of the Second Supplementary Notes 1 to 12,

in which the microorganism is a hydrogen-oxidizing bacterium,
the raw material gas contains hydrogen as the combustible gas and contains oxygen and carbon dioxide as other components, and
the adjustment gas is carbon dioxide.

[Second Supplementary Note 14]

The culture device according to any one of the Second Supplementary Notes 1 to 13, further comprising:
a first component separation mechanism that separates a specific component from a mixed gas taken out from the culture tank, or a second component separation mechanism that separates a specific component from a separation gas separated from the culture solution taken out from the culture tank.

[Second Supplementary Note 15]

The culture device according to any one of the Second Supplementary Notes 11 to 14, further comprising:

a gas circulation path for reusing the mixed gas in the space as the raw material gas; and
a dissolution rate control mechanism that measures, during the culture, a combustible gas dissolution rate, which is a proportion of the combustible gas dissolved in the culture solution, to the combustible gas contained in the raw material gas supplied to the culture tank through the gas circulation path and the raw material gas supply unit, and maintains the measured combustible gas dissolution rate at a preset target value,
in which, in the gas circulation path, a mixed gas extraction port that takes out the mixed gas from the culture tank is disposed at a position allowing a foam layer present on the liquid surface in the culture tank to be taken in.

[Second Supplementary Note 16]

The culture device according to any one of the Second Supplementary Notes 1 to 15, further comprising:

a culture solution recovery mechanism that recovers a recovery solution, which is a part of the culture solution containing the produced organic substance, from the culture solution taken out from the culture tank;
a recovery solution tank that stores the recovery solution containing the organic substance; and
a concentration reduction mechanism that reduces a dissolved combustible gas concentration in the recovery solution stored in the recovery solution tank to 1/10 or less of a dissolved combustible gas concentration in the culture solution in the culture tank.

[Second Supplementary Note 17]

The culture device according to any one of the Second Supplementary Notes 1 to 16, further comprising:
an explosion suppression mechanism that suppresses an explosion in a gas reuse path for reusing a gas separated from the culture solution.

[Second Supplementary Note 18]

A method for controlling a culture device that cultures a microorganism or a cell and that includes a culture tank that accommodates a culture solution for culturing a microorganism or a cell that uses a raw material gas containing a combustible gas for proliferation or production of an organic substance, and a raw material gas supply unit that supplies the raw material gas containing a combustible gas to the culture tank, in which, in a case where a circular equivalent diameter of a cross section orthogonal to a height direction of the culture tank is defined as DAm and a maximum height of the liquid surface is defined as H, a dimension of the culture tank satisfies a condition of $H/DAm \geq 2$, the method including: measuring a composition ratio of a mixed gas in a space above a liquid surface of the culture solution in the culture tank; and supplying an adjustment gas into the space in accordance with the measured composition ratio of the mixed gas to maintain the composition ratio of the mixed gas in the space at a target value outside a preset explosive range.

(Third Supplementary Notes)

[Third Supplementary Note 1]

A culture device that cultures a microorganism or a cell that uses a raw material gas containing a combustible gas for proliferation or production of an organic substance, the culture device comprising:

a culture tank that accommodates a culture solution for culturing the microorganism or the cell;
a culture solution tank that stores the culture solution to be supplied to the culture tank;
a culture target supply path that supplies the microorganism or the cell to the culture tank;
a raw material gas tank that stores the raw material gas;
a bubble generation device for mixing the raw material gas with the culture solution before the culture solution is supplied to the culture tank; and
a culture solution supply path that is disposed between the culture solution tank and the culture tank, and supplies the culture solution mixed with the raw material gas by the bubble generation device to the culture tank, the culture solution supply path being provided separately from the culture target supply path.

[Third Supplementary Note 2]

The culture device according to the Third Supplementary Note 1,
in which a temperature adjuster that adjusts a temperature of the culture solution is provided in each of the culture tank and the culture solution tank.

[Third Supplementary Note 3]

The culture device according to the Third Supplementary Note 1 or 2,
in which the bubble generation device is provided in the culture solution tank.

[Third Supplementary Note 4]

The culture device according to any one of the Third Supplementary Notes 1 to 3,
in which the bubble generation device generates bubbles by a vortex shear force.

[Third Supplementary Note 5]

The culture device according to the Third Supplementary Notes 1 to 4, further comprising:
a culture solution circulation path that takes out a part of the culture solution from the culture tank, recovers a part of the microorganism or the cell from the taken-out culture solution, and returns a remaining culture solution after the recovery to the culture tank through the culture solution tank.

[Third Supplementary Note 6]

The culture device according to any one of the Third Supplementary Notes 1 to 5,

in which the microorganism is a hydrogen-oxidizing bacterium, and
the raw material gas contains hydrogen as the combustible gas and contains oxygen and carbon dioxide as other components.

[Third Supplementary Note 7]

The culture device according to the Third Supplementary Note 6,
in which, as the raw material gas, each component of hydrogen, oxygen, and carbon dioxide is supplied separately.

[Third Supplementary Note 8]

The culture device according to any one of the Third Supplementary Notes 1 to 7,
in which the d90 of a bubble diameter of the raw material gas generated in the culture solution is 1000 $\mu$m or less.

[Third Supplementary Note 9]

The culture device according to any one of the Third Supplementary Notes 1 to 8, further comprising:
a composition ratio control mechanism that measures a composition ratio of a mixed gas in a space above a liquid surface of the culture solution in the culture tank, and supplies an adjustment gas into the space in accordance with the measured composition ratio of the mixed gas to maintain the composition ratio of the mixed gas in the space at a target value outside a preset explosive range.

[Third Supplementary Note 10]

The culture device according to any one of the Third Supplementary Notes 1 to 9, further comprising:
a first component separation mechanism that separates a specific component from a mixed gas taken out from the culture tank, or a second component separation mechanism that separates a specific component from a separation gas separated from the culture solution taken out from the culture tank.

[Third Supplementary Note 11]

The culture device according to any one of the Third Supplementary Notes 1 to 10,
in which, in a gas circulation path for circulating a mixed gas taken out from the culture tank, a mixed gas extraction port that takes out the mixed gas from the culture tank is disposed at a position allowing a foam layer present on a liquid surface of the culture solution in the culture tank to be taken in.

[Third Supplementary Note 12]

The culture device according to any one of the Third Supplementary Notes 1 to 11, further comprising:

a culture solution recovery mechanism that recovers a recovery solution, which is a part of the culture solution containing the produced organic substance, from the culture solution taken out from the culture tank;
a recovery solution tank that stores the recovery solution containing the organic substance; and
a concentration reduction mechanism that reduces a dissolved combustible gas concentration in the recovery solution stored in the recovery solution tank to 1/10 or less of a dissolved combustible gas concentration in the culture solution in the culture tank.

[Third Supplementary Note 13]

The culture device according to any one of the Third Supplementary Notes 1 to 12, further comprising:
an explosion suppression mechanism that suppresses an explosion in a gas reuse path for reusing a gas separated from the culture solution.

(Fourth Supplementary Notes)

[Fourth Supplementary Note 1]

A culture device that cultures a microorganism or a cell that uses a raw material gas containing a combustible gas for proliferation or production of an organic substance, the culture device comprising: a culture tank that accommodates a culture solution for culturing the microorganism or the cell; a raw material gas supply unit that supplies the raw material gas to the culture tank; a gas circulation path for reusing a mixed gas in a space above a liquid surface of the culture solution in the culture tank as the raw material gas; and a pressure applying device that is disposed on the gas circulation path and applies a circulation pressure to the mixed gas to circulate the mixed gas, in which the pressure applying device does not have a mechanical movable part, and applies the circulation pressure to the mixed gas by a drive fluid input to the pressure applying device.

[Fourth Supplementary Note 2]

The culture device according to the Fourth Supplementary Note 1,
in which the pressure applying device is a fluid ejector.

[Fourth Supplementary Note 3]

The culture device according to the Fourth Supplementary Note 2,
in which the drive fluid input to the fluid ejector is the culture solution taken out from the culture tank.

[Fourth Supplementary Note 4]

The culture device according to any one of the Fourth Supplementary Notes 1 to 3,

in which, during the culture, a consumption amount of a raw material gas consumed by the microorganism or the cell in the raw material gas supplied to the culture tank is measured, and
the raw material gas supply unit supplies the raw material gas in an amount equal to or greater than the consumption amount of the raw material gas.

[Fourth Supplementary Note 5]

The culture device according to any one of the Fourth Supplementary Notes 1 to 4,
in which a supply amount of the raw material gas supplied to the culture tank through the raw material gas supply unit and the gas circulation path is 2 to 15 times a consumption amount of the raw material gas.

[Fourth Supplementary Note 6]

The culture device according to any one of the Fourth Supplementary Notes 1 to 5, further comprising:
a composition ratio control mechanism that measures a composition ratio of a mixed gas in a space above a liquid surface of the culture solution in the culture tank, and supplies an adjustment gas into the space in accordance with the measured composition ratio of the mixed gas to maintain the composition ratio of the mixed gas in the space at a target value outside a preset explosive range.

[Fourth Supplementary Note 7]

The culture device according to any one of the Fourth Supplementary Notes 1 to 6,
in which, in the gas circulation path, a mixed gas extraction port that takes out the mixed gas from the culture tank is disposed at a position allowing a foam layer present on the liquid surface in the culture tank to be taken in.

[Fourth Supplementary Note 8]

The culture device according to any one of the Fourth Supplementary Notes 1 to 7,

in which the microorganism is a hydrogen-oxidizing bacterium, and
the raw material gas contains hydrogen as the combustible gas and contains oxygen and carbon dioxide as other components.

[Fourth Supplementary Note 9]

The culture device according to any one of the Fourth Supplementary Notes 1 to 8, further comprising:
a first component separation mechanism that separates a specific component from a mixed gas taken out from the culture tank, or a second component separation mechanism that separates a specific component from a separation gas separated from the culture solution taken out from the culture tank.

[Fourth Supplementary Note 10]

The culture device according to any one of the Fourth Supplementary Notes 1 to 9, further comprising:

a culture solution recovery mechanism that recovers a recovery solution, which is a part of the culture solution containing the produced organic substance, from the culture solution taken out from the culture tank;
a recovery solution tank that stores the recovery solution containing the organic substance; and
a concentration reduction mechanism that reduces a dissolved combustible gas concentration in the recovery solution stored in the recovery solution tank to 1/10 or less of a dissolved combustible gas concentration in the culture solution in the culture tank.

[Fourth Supplementary Note 11]

The culture device according to any one of the Fourth Supplementary Notes 1 to 10, further comprising:
an explosion suppression mechanism that suppresses an explosion in a gas reuse path for reusing a gas separated from the culture solution.

The processor described above includes, in addition to a general-purpose processor such as a CPU that functions as various processing units by executing software (program), a programmable logic device (PLD) which is a processor capable of changing a circuit configuration after manufacture such as a field programmable gate array (FPGA), a dedicated electric circuit which is a processor having a circuit configuration exclusively designed to execute specific processing such as an application specific integrated circuit (ASIC), and the like.

Furthermore, as a hardware structure of the various processors, more specifically, an electric circuitry in which circuit elements such as semiconductor elements are combined can be used.

The above-described contents and illustrated contents are detailed descriptions of parts related to the technology of the present disclosure, and are merely examples of the technology of the present disclosure. For example, the above descriptions related to configurations, functions, operations, and advantageous effects are descriptions related to examples of configurations, functions, operations, and advantageous effects of the parts related to the technology of the present disclosure. Therefore, it is needless to say that unnecessary parts may be deleted, or new elements may be added or replaced with respect to the above-described contents and illustrated contents within a scope not departing from the spirit of the technology of the present disclosure. In order to avoid complications and easily understand the parts according to the technology of the present disclosure, in the above-described contents and illustrated contents, common technical knowledge and the like that do not need to be described to implement the technology of the present disclosure are not described.

The disclosure of JP2023-125057 filed on July 31, 2023, is incorporated in the present specification by reference. In addition, all documents, patent applications, and technical standards described in the present specification are incorporated in the present specification by reference to the same extent as in a case where each document, patent application, and technical standard are specifically and individually noted to be incorporated by reference.

## Claims

1. A culture device that cultures a microorganism or a cell that uses a raw material gas containing a combustible gas for proliferation or production of an organic substance, the culture device comprising:

   a culture tank that accommodates a culture solution for culturing the microorganism or the cell;
   a raw material gas supply unit that supplies the raw material gas to the culture tank; and
   a composition ratio control mechanism that measures a composition ratio of a mixed gas in a space above a liquid surface of the culture solution in the culture tank and supplies an adjustment gas into the space in accordance with the measured composition ratio of the mixed gas to maintain the composition ratio of the mixed gas in the space at a target value outside a preset explosive range,
   wherein, in a case where a circular equivalent diameter of a cross section orthogonal to a height direction of the culture tank is defined as DAm and a maximum height of the liquid surface is defined as H, a dimension of the culture tank satisfies a condition of $H/DAm \geq 2$.

2. The culture device according to claim 1,
   wherein the culture tank has a bottomed cylindrical shape.

3. The culture device according to claim 1,
wherein the condition is H/DAm ≥ 13.

4. The culture device according to claim 1,
wherein d90 of a bubble diameter of the raw material gas generated in the culture solution is 1000 μm or less.

5. The culture device according to claim 1, further comprising:

a temperature adjuster that adjusts a temperature of the culture solution,
wherein at least a part of the temperature adjuster is disposed in the culture tank.

6. The culture device according to claim 5,
wherein a dimension of the temperature adjuster in the height direction of the culture tank is a length of 30% or more of the maximum height of the liquid surface in total.

7. The culture device according to claim 5,
wherein a plurality of temperature adjusters are provided, and each of the plurality of temperature adjusters is disposed at a different position in the height direction of the culture tank.

8. The culture device according to claim 5, further comprising:

a stirring mechanism that includes a plurality of stirring units that stir the culture solution,
wherein the plurality of stirring units are disposed at different positions in the height direction of the culture tank.

9. The culture device according to claim 8,
wherein, in a case where a plurality of temperature adjusters and the plurality of stirring units are provided, a plurality of pairs, each including at least one temperature adjuster and at least one stirring unit, are disposed at different positions in the height direction of the culture tank.

10. The culture device according to claim 1, further comprising:

a gas circulation path for reusing the mixed gas in the space as the raw material gas; and
a dissolution rate control mechanism that measures, during the culture, a combustible gas dissolution rate, which is a proportion of the combustible gas dissolved in the culture solution, to the combustible gas contained in the raw material gas supplied to the culture tank through the gas circulation path and the raw material gas supply unit, and maintains the measured combustible gas dissolution rate at a preset target value.

11. The culture device according to claim 10,
wherein the target value of the combustible gas dissolution rate is 90% or more.

12. The culture device according to claim 10,
wherein the dissolution rate control mechanism further measures, during the culture, a combustible gas consumption rate of the combustible gas consumed by the microorganism or the cell to the combustible gas contained in the raw material gas supplied to the culture tank through the gas circulation path or the raw material gas supply unit, and maintains the measured combustible gas consumption rate at 90% or more through the control of the combustible gas dissolution rate.

13. The culture device according to claim 1,

wherein the microorganism is a hydrogen-oxidizing bacterium,
the raw material gas contains hydrogen as the combustible gas and contains oxygen and carbon dioxide as other components, and
the adjustment gas is carbon dioxide.

14. The culture device according to claim 1, further comprising:
a first component separation mechanism that separates a specific component from the mixed gas taken out from the culture tank, or a second component separation mechanism that separates a specific component from a separation gas separated from the culture solution taken out from the culture tank.

**15.** The culture device according to claim 10,
wherein, in the gas circulation path, a mixed gas extraction port that takes out the mixed gas from the culture tank is disposed at a position allowing a foam layer present on the liquid surface in the culture tank to be taken in.

**16.** The culture device according to claim 1, further comprising:

a culture solution recovery mechanism that recovers a recovery solution, which is a part of the culture solution containing the produced organic substance, from the culture solution taken out from the culture tank;
a recovery solution tank that stores the recovery solution containing the organic substance; and
a concentration reduction mechanism that reduces a dissolved combustible gas concentration in the recovery solution stored in the recovery solution tank to 1/10 or less of a dissolved combustible gas concentration in the culture solution in the culture tank.

**17.** The culture device according to claim 1, further comprising:
an explosion suppression mechanism that suppresses an explosion in a gas reuse path for reusing a gas separated from the culture solution.

**18.** A method for controlling a culture device that cultures a microorganism or a cell and that includes a culture tank that accommodates a culture solution for culturing a microorganism or a cell that uses a raw material gas containing a combustible gas for proliferation or production of an organic substance, and a raw material gas supply unit that supplies the raw material gas containing a combustible gas to the culture tank, in which, in a case where a circular equivalent diameter of a cross section orthogonal to a height direction of the culture tank is defined as DAm and a maximum height of the liquid surface is defined as H, a dimension of the culture tank satisfies a condition of H/DAm $\geq$ 2, the method comprising:

measuring a composition ratio of a mixed gas in a space above a liquid surface of the culture solution in the culture tank; and
supplying an adjustment gas into the space in accordance with the measured composition ratio of the mixed gas to maintain the composition ratio of the mixed gas in the space at a target value outside a preset explosive range.

# FIG. 1

RAW MATERIAL GAS

H₂
CO₂
O₂
…

CULTURE DEVICE 10

OF MICROORGANISMS (HYDROGEN-OXIDIZING BACTERIA)

PRODUCTION OF ORGANIC SUBSTANCE (ALCOHOL, AMINO ACIDS)

PURIFICATION

ORGANIC SUBSTANCE (ALCOHOL, AMINO ACIDS)

SEPARATION OF MICROORGANISMS

MICROORGANISMS (HYDROGEN-OXIDIZING BACTERIA)

USE AS FEED, FOOD, FUEL, OR THE LIKE

EP 4 733 379 A1

FIG. 2

# FIG. 3

# FIG. 4

# FIG. 5

# FIG. 6

# FIG. 7

## FIG. 8

SURFACE TENSION
REDUCER

13

18

CULTURE SOLUTION

SOLUBILITY OF RAW MATERIAL
GAS CAN BE IMPROVED

## FIG. 9

PLANT

WASTE GAS
OF PLANT

52

12

RAW
26 MATERIAL GAS

$H_2$

$CO_2$

22    23

P

12A

$O_2$

$CO_2$

⋮

# FIG. 10

CONTROL SIGNAL ← PROCESSOR ── 17

MEASUREMENT SIGNAL/DETECTION SIGNAL →

17A ─ MEMORY

CULTURE CONDITION INFORMATION

· TARGET TEMPERATURE OF CULTURE SOLUTION
· TARGET PRESSURE IN SPACE

· TARGET VALUE $(TD(H_2)$, $(TD(O_2))$ OUTSIDE EXPLOSION RANGE OF COMPOSITION RATIO OF MIXED GAS IN SPACE

· TARGET VALUE OF HYDROGEN DISSOLUTION RATE: $(TR_d(H_2))$

· TARGET VALUE OF HYDROGEN CONSUMPTION RATE: $(TR_c(H_2))$

# FIG. 11

# FIG. 12

HYDROGEN DISSOLUTION RATE CONTROL: $Rd(H_2) \geq TRd(H_2)$

$\longrightarrow$ REDUCED

$$Rd(H_2) = (1 - Qout(H_2) / Qin(H_2)) \times 100$$

$$Qout(H_2) = Qout(total) \times D(H_2)$$

HYDROGEN CONSUMPTION RATE CONTROL: $Rc(H_2) \geq TRc(H_2)$

$\longrightarrow$ REDUCED

$$Rc(H_2) = Qcs(H_2) / Qin(H_2) \times 100$$

$$Qcs(H_2) = Qin(H_2) - (Qout(H_2) + Qr(H_2))$$

$$Qr(H_2) = Qm \times Dr(H_2)$$

## FIG. 13

COMPOSITION RATIO CONTROL PROCESS

S101 — ACQUIRE COMPOSITION RATIO OF MIXED GAS

S102 — IS COMPOSITION RATIO OUTSIDE EXPLOSION RANGE? — Y

N

S103 — SUPPLY ADJUSTMENT GAS

S104 — IS CULTURE PROCESS COMPLETED?

N

Y

END

## FIG. 14

DISSOLUTION RATE CONTROL PROCESS

S201 — MEASURE HYDROGEN DISSOLUTION RATE $R_d(H_2)$

S202 — HYDROGEN DISSOLUTION RATE $R_d(H_2) \geq$ TARGET VALUE $TR_d(H_2)$ — Y

N

S203 — REDUCE HYDROGEN SUPPLY AMOUNT $Q_{in}(H_2)$

S204 — IS CULTURE PROCESS COMPLETED?

N

Y

END

## FIG. 15

CONSUMPTION RATE CONTROL PROCESS

S301
MEASURE HYDROGEN CONSUMPTION RATE $Rc(H_2)$

S302
HYDROGEN CONSUMPTION RATE $Rc(H_2) \geq$ TARGET VALUE $TRc(H_2)$ — Y

N

S303
REDUCE HYDROGEN SUPPLY AMOUNT $Qin(H_2)$

S304
IS CULTURE PROCESS COMPLETED? — N

Y

END

# FIG. 16

```
┌─────────────────────────────────────────────────────┐
│  ┌────────────────────────────────────────────────┐ │
│  │ 〈HYDROGEN DISSOLUTION RATE CONTROL PROCESS〉    │ │
│  │                                                 │ │
│  │ MAINTAIN HYDROGEN DISSOLUTION RATE EQUAL TO OR  │ │
│  │        HIGHER THAN TARGET VALUE                 │ │
│  └────────────────────────────────────────────────┘ │
│  ┌────────────────────────────────────────────────┐ │
│  │ 〈HYDROGEN CONSUMPTION RATE CONTROL PROCESS〉    │ │
│  │                                                 │ │
│  │  MAINTAIN HYDROGEN CONSUMPTION RATE EQUAL TO    │ │
│  │        OR HIGHER THAN TARGET VALUE              │ │
│  └────────────────────────────────────────────────┘ │
└─────────────────────────────────────────────────────┘
```

AMOUNT OF HYDROGEN CONTAINED IN MIXED GAS IS REDUCED

SAFETY IS IMPROVED

UTILIZATION EFFICIENCY OF RAW MATERIAL GAS IS IMPROVED

SUPPLY AMOUNT REDUCTION OF ADJUSTMENT GAS WHEN COMPOSITION RATIO CONTROL IS PERFORMED

# FIG. 17

DIMENSION CONDITION
OF CULTURE TANK:
$H/DAm \geq 2$
PREFERABLY
$H/DAm \geq 13$

DIMENSION CONDITION
OF TEMPERATURE
ADJUSTER:
$Ht \geq H \times 0.3$

CULTURE
SOLUTION

# FIG. 18

## FIG. 19

# FIG. 20

# FIG. 21

FIG. 22

EP 4 733 379 A1

FIG. 23

EP 4 733 379 A1

# FIG. 24

410

CULTURE TARGET RECOVERY

## INTERNATIONAL SEARCH REPORT

| International application No. |
|---|
| **PCT/JP2024/024284** |

| **A. CLASSIFICATION OF SUBJECT MATTER** |
|---|
| *C12M 1/04*(2006.01)i; *C12M 1/00*(2006.01)i; *C12N 1/00*(2006.01)i<br>FI: C12M1/04: C12M1/00 D; C12N1/00 B |
| According to International Patent Classification (IPC) or to both national classification and IPC |

| **B. FIELDS SEARCHED** |
|---|
| Minimum documentation searched (classification system followed by classification symbols)<br>C12M1/04; C12M1/00; C12N1/00; B01D53/34 |
| Documentation searched other than minimum documentation to the extent that such documents are included in the fields searched<br>Published examined utility model applications of Japan 1922-1996<br>Published unexamined utility model applications of Japan 1971-2024<br>Registered utility model specifications of Japan 1996-2024<br>Published registered utility model applications of Japan 1994-2024 |
| Electronic data base consulted during the international search (name of data base and, where practicable, search terms used) |

| **C. DOCUMENTS CONSIDERED TO BE RELEVANT** | | |
|---|---|---|
| Category* | Citation of document, with indication, where appropriate, of the relevant passages | Relevant to claim No. |
| X | JP 06-165686 A (SEIBU GAS KK) 14 June 1994 (1994-06-14)<br>claims 1, paragraphs [0006]-[0011], example 1, fig. 1 | 1-7, 13, 17-18 |
| Y | claims 1, paragraphs [0006]-[0011], example 1, fig. 1 | 8-12, 14-16 |
| Y | JP 2009-072133 A (HITACHI PLANT TECHNOLOGIES, LTD.) 09 April 2009 (2009-04-09)<br>claims, fig. 1 | 8-9 |
| Y | JP 04-356196 A (SHIN-ETSU CHEMICAL CO., LTD.) 09 December 1992 (1992-12-09)<br>claims, fig. 1 | 8-9 |
| Y | JP 03-127983 A (SEIBU GAS KK) 31 May 1991 (1991-05-31)<br>claims | 10-12, 15 |
| Y | JP 2010-537822 A (CLEARVALUE TECHNOLOGIES, INC.) 09 December 2010<br>(2010-12-09)<br>claims 51, 54, 58, 60 | 14, 16 |

☑ Further documents are listed in the continuation of Box C.    ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | "&" document member of the same patent family |
| "P" document published prior to the international filing date but later than the priority date claimed | |

| Date of the actual completion of the international search | Date of mailing of the international search report |
|---|---|
| **15 August 2024** | **03 September 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

**INTERNATIONAL SEARCH REPORT**

International application No.

**PCT/JP2024/024284**

**C.    DOCUMENTS CONSIDERED TO BE RELEVANT**

| Category* | Citation of document, with indication, where appropriate, of the relevant passages | Relevant to claim No. |
|---|---|---|
| P, A | WO 2024/048796 A1 (FUJIFILM CORPORATION) 07 March 2024 (2024-03-07)<br>entire text | 1-18 |

Form PCT/ISA/210 (second sheet) (July 2022)

**INTERNATIONAL SEARCH REPORT**
Information on patent family members

International application No.

**PCT/JP2024/024284**

| Patent document cited in search report | | | Publication date (day/month/year) | Patent family member(s) | | | Publication date (day/month/year) |
|---|---|---|---|---|---|---|---|
| JP | 06-165686 | A | 14 June 1994 | (Family: none) | | | |
| JP | 2009-072133 | A | 09 April 2009 | US<br>claims, fig. 1<br>EP | 2009/0081723<br><br>2039754 | A1<br><br>A1 | |
| JP | 04-356196 | A | 09 December 1992 | (Family: none) | | | |
| JP | 03-127983 | A | 31 May 1991 | (Family: none) | | | |
| JP | 2010-537822 | A | 09 December 2010 | WO<br>claims 51, 54, 58, 60<br>EP<br>US | 2009/032331<br><br>2185270<br>2009/0087898 | A2<br><br>A2<br>A1 | |
| WO | 2024/048796 | A1 | 07 March 2024 | (Family: none) | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2564008 B
- US 20190316072 A
- WO 2011070791 A
- JP 2013005754 A
- WO 2006101074 A
- WO 2016117023 A
- JP 6528295 B
- JP 2023125057 A